# EUROPEAN PATENT APPLICATION

(11) **EP 3 693 740 A1**
(43) Date of publication of application: **12.08.2020**
(21) Application number: 19155958.2
(22) Date of filing: 07.02.2019
(51) Int. Cl.: G01N 33/574, A61K 47/69

(54) **IMPROVED DITHIOCARMABATE BASED COMPOUNDS, THERAPIES AND DIAGNOSTICS**

(71) Applicant: Deutsches Krebsforschungszentrum, Stiftung des öffentlichen Rechts, 69120 Heidelberg (DE)
(72) Inventor: Gunkel, Nikolas, 69121 Heidelberg (DE); Amtmann, Eberhard, 69121 Heidelberg (DE); Morgen, Michael, 56812 Valwig (DE); Miller, Aubry, 69126 Heidelberg (DE)
(74) Representative: Kuttenkeuler, David

(57) **Abstract**

The present invention provides an improved class of dithiocarbamate complexes with increased tumour spheroid activity. The invention provides the novel compounds as such, and their medical application for the treatment of tumour diseases. The compounds of the invention are surprisingly effective compared to the prior art dithiocarbamates, in particular with respect to tumour spheroids. Another aspect of the herein described invention provides a diagnostic determination of tumours with increased susceptibility to a treatment with the dithiocarbamates of the invention. This aspect of the invention suggests determining components of the thioredoxin 1 (TRX1)/thioredoxin reductase 1 (TRXR1) system as an indicator of treatment responders. In context of this inventive aspect, the dithiocarbamate compounds of the invention were identified as competitive TRXR1 binders, and hence, the TRX1-amount in tumours is an important factor for treatment success.

## Description

### FIELD OF THE INVENTION

The present invention provides an improved class of dithiocarbamate complexes with increased tumour spheroid activity. The invention provides the novel compounds as such, and their medical application for the treatment of tumour diseases. The compounds of the invention are surprisingly effective compared to the prior art dithiocarbamates, in particular with respect to tumour spheroids. Another aspect of the herein described invention provides a diagnostic determination of tumours with increased susceptibility to a treatment with the dithiocarbamates of the invention. This aspect of the invention suggests determining components of the thioredoxin 1 (TRX1)/thioredoxin reductase 1 (TRXR1) system as an indicator of treatment responders. In context of this inventive aspect, the dithiocarbamate compounds of the invention were identified as competitive TRXR1 binders, and hence, the TRX1-amount in tumours is an important factor for treatment success.

### DESCRIPTION

Cancer, the uncontrolled growth of malignant cells, is a major health problem of the modern medical era. While some malignancies, such as adenocarcinoma of the breast and lymphomas such as Hodgkin's Disease, respond relatively well to current chemotherapeutic antineoplastic drug regimens, other cancers are poorly responsive to chemotherapy, especially melanoma, non-small cell lung, pancreatic, liver, prostate and colon cancers. Even small cell cancer of the lung, initially chemotherapy sensitive, tends to return after remission, with widespread metastatic spread leading to death of the patient. Thus, better treatment approaches are needed for these illnesses.

Small cell lung cancer (SCLC) is still an intractable cancer as defined by its five-year relative survival rate of less than 7 percent and an estimated annual loss of 250,000 lives worldwide. Initially, SCLC was characterized as unusually chemo- and radio-sensitive on the basis of the early clinical trials, which led to optimism concerning the curability of SCLC. Indeed, the current first-line treatment regimen for SCLC in most countries is a combination of etoposide and cisplatin (EP), typically resulting in a remarkable response. The clinical reality, however, shows that the median survival time for patients with limited-stage disease is approximately 18 months, while survival time for the two-thirds of patients who are diagnosed with more advanced disease is 9 months (Nicholson et al., J Thorac Oncol. 2016 Mar;11(3):300-11. doi: 10.1016/j.jtho.2015.10.008.). This is primarily due to a rapid occurrence of therapy-resistant cells, leaving most patients with only palliative options.

Cigarette smoke, the main cause of lung cancer, contains a considerable amount of toxic chemicals, including reactive nitrogen species (RNS), reactive oxygen species (ROS), and free radicals. As such, it is not surprising that SCLC tumours typically contain a large number of genetic alterations (Bunn et al.,; J Thorac Oncol. 2016 Apr;11(4):453-74. doi: 10.1016/j.jtho.2016.01.012.). Recent pharmacogenomic approaches using cell line collections and patient material provided a detailed list of potential SCLC drug target candidates and suggested that off-label use of some approved drugs could be beneficial (Polley et al., J Natl Cancer Inst. 2016 May 31;108(10). doi: 10.1093/jnci/djw122. Print 2016 Oct.). However, further downstream in the process of SCLC-drug development, at the level of clinical trials, none of the more than 25 different targeted therapy drugs have shown more than only limited benefits for patients (Koinis et al., J Thorac Oncol. 2016 Aug;11(8):1263-1272. doi: 10.1016/j.jtho.2016.04.026.). These outcomes reflect high tumour heterogeneity in SCLC and also the poor health status of most SCLC patients, which limits options due to the dose-limiting toxicities of any potential SCLC drug.

Because patients tend to initially respond well to EP, it is logical to seek a maintenance therapy rather than a replacement of first line therapy in SCLC. A treatment concept that significantly prolongs the survival time of SCLC patients would need to overcome therapy resistance of residual cancer cells after first line therapy, and would need to be tolerable for long-term treatment to enable the maintenance of the first line therapy effect. The efficacy of such a selective drug would be a function of the vulnerability of the treated tumour, the inability of the tumour to develop cross resistance mechanisms during the cytotoxic assault by the drug and conversely, the availability of robust mechanisms in healthy tissue to defend against drug effects.

Dithiocarbamates and also thiuram disulfides such as tetraethylthiuram disulfide, hereinafter also called disulfiram, are chelators of, or react with, heavy metals and are used as a therapeutic agents.. Disulfiram for example is the active ingredient in the drug Antabuse® used for many years in aversion therapy for chronic alcoholism. Disulfiram also has the potential to be used as a treatment for neoplastic diseases (Wickstrom, Danielsson et al. (Biochem Pharmacol. 2007 Jan 1;73(1):25-33. Epub 2006 Aug 26.), Conticello, Martinetti et al. (Int J Cancer. 2012 Nov 1;131(9):2197-203. doi: 10.1002/ijc.27482. Epub 2012 Mar 27.), Triscott, Rose Pambid et al. (Stem Cells. 2015 Apr;33(4):1042-6. doi: 10.1002/stem.1956), Skrott et al., 2017 (Nature, Dec 14;552(7684):194-199. doi: 10.1038/nature25016)). U.S. Patent No. 6,288,1 10, entitled, "Pharmaceutical compositions comprising disulfiram," discloses disulfiram to inhibit angiogenesis and to be useful in the treatment of angiogenesis-dependent disorders, including neoplasms, and to prevent cell hyperproliferation and formation of clots along or around medical devices. The entire contents of which are incorporated herein by reference.

Phenotypically, disulfiram induces apoptosis, (Wang, Zhai et al, Cancer Lett. 2011 Jan 1;300(1):87-95. doi: 10.1016/j.canlet.2010.09.010. Epub 2010 Oct 29.) inhibits cell proliferation, and reduces angiogenesis, (Shian, Kao et al; Mol Pharmacol. 2003 Nov;64(5):1076-84.) thereby reducing invasion and metastasis. (Hothi, Martins et al.; Oncotarget. 2012 Oct;3(10):1124-3). The molecular mechanisms underlying these phenotypic effects are complex as disulfiram appears to inhibit multiple targets (Skrott et al., Nature. 2017 Dec 14;552(7684):194-199. doi: 10.1038/nature25016.). This might be the reason why disulfiram is effective in heterogeneous preclinical models like breast, prostate, myeloma, leukemia, melanoma, neuroblastoma, colorectal cancer, cervix adenocarcinoma and lung cancers.

WO 2018/069525 A1 describes a novel class or structure of dihiocarbamate-metal complexes and their uses in medicine which are described to have an improved anti-tumour effect, when combined with a cyclodextrin.

The invention intends to solve the following objects. Firstly, the invention seeks to provide novel diagnostic means to select patients that will likely benefit from a treatment with a dithiocarbamate compound according to the invention. Such diagnostics are intended to improve also the dosing regimen of the compound and the treatment plan in general. Secondly, the invention seeks to provide an improved class of dithiocarbamate compounds which are applicable for the treatment of patients suffering from tumour diseases, preferably such tumour diseases which are identified as susceptible to a dithiocarbamate treatment in accordance with the invention. Finally, the invention seeks to provide novel areas of use of the herein disclosed novel class of dithiocarbamate compounds.

Before the present invention is described, it is to be understood that this invention is not limited to particular embodiments described, and as such may, of course, vary. It is also to be understood that the terminology used herein is for the purpose of describing particular embodiments only, and is not intended to be limiting, since the scope of the present invention will be limited only by the appended claims.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

One or more of the herein disclosed objects of the invention are solved in a first aspect by a method for diagnosing a tumour disease in a patient, the method comprising the steps of
(a) Detecting the level of thioredoxin 1 (TRX1) in a sample from the patient;
(b) Comparing the detected level of TRX1 in the sample from the patient with a reference value or with a level of TRX1 in a reference sample,
wherein a reduced level of TRX1 in the sample from the patient compared to the reference value or with the level of TRX1 in a reference sample indicates a tumour which is treatable with a competitive thioredoxin reductase 1 (TRXR1) inhibitor.

Within the first aspect of the invention as an alternative there is provided a method for deciding how to treat a tumour disease in a patient, the method comprising the steps of
(a) Detecting the level of thioredoxin 1 (TRX1) in a sample from the patient;
(b) Comparing the detected level of TRX1 in the sample from the patient with a reference value or with a level of TRX1 in a reference sample,
wherein a reduced level of TRX1 in the sample from the patient compared to the reference value or with the level of TRX1 in a reference sample indicates a treatment of the patient with a competitive thioredoxin reductase 1 (TRXR1) inhibitor.

Within the first aspect of the invention, as an alternative, there is provided a method for stratifying a patient suffering from a cancer disease into a responder or non-responder group, wherein a responder refers to a patient which responds to a treatment with a competitive TRXR1 binder, such as a compound as disclosed herein, the method comprising the steps of
(a) Detecting the level of thioredoxin 1 (TRX1) in a sample from the patient;
(b) Comparing the detected level of TRX1 in the sample from the patient with a reference value or with a level of TRX1 in a reference sample,
wherein a reduced level of TRX1 in the sample from the patient compared to the reference value or with the level of TRX1 in a reference sample indicates that the patient is a responder of a treatment with a competitive thioredoxin reductase 1 (TRXR1) inhibitor.

In context of the herein disclosed invention it was discovered, that thioredoxin reductase 1 (TRXR1), when inhibited competitively, is a promising drug target for SCLC. DKFZ-608, a homoleptic dithiocarbamate gold complex, reversibly binds TRXR1 in competition with the TRXR1 substrate thioredoxin-1 (TRX1). Consequently, cells expressing low levels of TRX1, like SCLC, are hypersensitive to DKFZ-608, while cells, which express higher levels of TRX1, are resistant. DKFZ-608 causes oxidative stress in the cytoplasm, but not in mitochondria, further contributing to its low toxicity profile. Finally, in an SCLC animal model, DKFZ-608 eradicates residual tumour cells surviving first line therapy and completely prevents tumour recurrence.

As used herein, a "subject" or "patient" includes all mammals, including without limitation humans, but also non-human primates such as cynomolgus monkeys. It also includes dogs, cats, horses, sheep, pigs, cattle, goats, cows, rabbits, pigs and rodents (such as mice and rats). It will be appreciated that a particularly preferred subject according to the invention is a human subject, such as a human suffering from (or at risk of suffering from) a disorder, disease or condition, for example a human patient.

The term "thioredoxin" in context of the present invention shall pertain to a protein expressed by the gene *TXN* in humans. The protein encoded by this gene acts as a homodimer and is involved in many redox reactions. The encoded protein is active in the reversible S-nitrosylation of cysteines in certain proteins, which is part of the response to intracellular nitric oxide. This protein is found in the cytoplasm. Two transcript variants encoding different isoforms have been found for this gene. Thioredoxin protein takes part in various redox reactions through the reversible oxidation of its active centre dithiol to a disulphide and catalyses dithiol-disulphide exchange reactions. Thioredoxin is also referred to as TRX or TRX1 in context of the present invention. Information about the TRX1 protein can be derived from, for example, UniProt database (accession number P10599 - https://www.uniprot.org/uniprot/P10599 - in the UniProt Database version of December 2018). The TXN gene can be obtained via the human genename database with the accession number HGNC:12435 ("www.genenames.org") in the database version of February 2019.

Thioredoxin reductase 1 (TRXR1) is the only enzyme known to catalyse the reduction of thioredoxin and hence is a central component in the TRX/TRXR1 system. Together with TRX1 and NADPH this system's most general description is as a method of forming reduced disulphide bonds in cells. Electrons are taken from NADPH via TRXR1 and are transferred to the active site of TRX1, which goes on to reduce protein disulphides or other substrates.

The term "thioredoxin reductase" or "TRXR" or "TRXR1", refers to a cytoplasmatic enzyme having 7 isoforms, which are all included by the present invention. The various isoforms harbour different biological functions but generally catalyse the reduction of TRX and therefore has a central role in the TRX system. The TRX system is extensively studied to date, for example as reviewed in (E. Arner Biochimica et Biophysica Acta 1790 (2009) 495-526, DOI: 10.1016/j.bbagen.2009.01.014). The TRXR1 protein and its multiple isoforms are provided in UniProt under the accession number Q16881 - https://www.uniprot.org/uniprot/Q16881 - in the database version of December 2018.

In certain embodiments, a "sample" is one obtained from a mammalian subject like a human patient. The term "biological sample" is used in its broadest sense and can refer to a bodily sample obtained from the subject (eg, a human patient). For example, the biological sample can include a clinical sample, i.e., a sample derived from a subject. Such samples can include, but are not limited to: peripheral bodily fluids, which may or may not contain cells, e.g., blood, urine, plasma, mucous, bile pancreatic juice, supernatant fluid, and serum; tissue or fine needle biopsy samples; tumour biopsy samples or sections (or cells thereof), and archival samples with known diagnosis, treatment and/or outcome history. Biological samples may also include sections of tissues, such as frozen sections taken for histological purposes. The term "biological sample" can also encompass any material derived by processing the sample. Derived materials can include, but are not limited to, cells (or their progeny) isolated from the biological sample, nucleic acids and/or proteins extracted from the sample. Processing of the biological sample may involve one or more of, filtration, distillation, extraction, amplification, concentration, fixation, inactivation of interfering components, addition of reagents, and the like. Preferred is in some embodiments of these detection, determination and/or diagnosis methods, that the biological sample is a tissue sample from the subject, such as a sample of a tumour or a cancer from the subject. As described above, such tissue sample may be a biopsy sample of the tumour or a cancer such as a needle biopsy samples, or a tumour biopsy sections or an archival sample thereof. Such a tissue sample may comprise living, dead or fixed cells, such as from the tumour or a cancer, and such cells may be suspected of expressing (e.g. aberrantly or localised) the applicable biomarker to be determined.

In some embodiments, determination and/or diagnosis according to a method of the invention can comprise, such as in a further step, comparing the detected amount of (eg protein or mRNA of) the applicable biomarker (eg TRX1) with a reference value, cut-off value or control sample; wherein a detected amount greater than the reference or cut-off value indicates, or stratifies a patient for, a treatment with a compound of the invention. Such a reference or cut-off value may be determined from the use of a control assay, or may be predetermined from one or more values obtained from a study or a plurality of samples derived from tumours previously successfully treated. For example, a cut-off value for a diagnostic test may be determined by the analysis of samples taken from patients in the context of a controlled clinical study, and determination of a cut-off depending on the desired (or obtained) sensitivity and/or specificity of the test. In accordance with the present invention there are various references that can be used. One embodiment pertains to a reference value which corresponds to the level of TRX1 in tumour which cannot be treated efficiently with a competitive TRXR1 binder of the invention. In this embodiment the reference indicates that if observed TRX1 levels in patient samples are lower, a treatment according to the invention is possible.

Examples of methods useful in the detection of (such as the presence or absence of, or an amount of) the applicable biomarker (such as TRX1) include immunoassays, such as the enzyme linked immunosorbent assay (ELISA) and the radioimmunoassay (RIA), which employ an antigen binding protein such as an antibody or an antigen-binding fragment thereof, that specifically binds to such applicable biomarker.

In context of the herein disclosed invention detecting the level of TRX1 in the sample involves detection of the level of protein and/or mRNA of TRX1, and/or the TRX1 promoter region methylation status. For example the methylation in the promoter region is detected at CpG sites at positions -995, and /or -1418 distal to the translational start site (ATG) in the TRX1 gene, or to a region not more than 100bp, 50bp, 40bp, or 20bp, preferably 10bp, most preferably 5bp away from positions -995, and /or -1418.

Alternatively, the presence of the applicable biomarker (eg TRX1) may be detected by detection of the presence of mRNA that encodes such applicable biomarker, or fragments of such mRNA. Methods to detect the presence of such mRNA (or fragments) can include, PCR (such as quantitative RT-PCR), hybridisation (such as to Illumina chips), nucleic-acid sequencing etc. Such methods may involve or comprise steps using one or more nucleic acids as described herein, such as PCR primers or PCR probes, or hybridisation probes, that bind (eg specifically) to such mRNA.

In preferred embodiments, the TRX1 promoter region comprises at least one response element for oxidative stress.

In preferred embodiments a patient or subject in context of the present invention is a mammalian subject, most preferably a human. In this context as mentioned before the TRX1 is preferably human TRX1, and wherein the TRXR1 is human TRXR1.

In preferred embodiments the methods of the invention are performed *in vitro* or *ex vivo*, or wherein the method does not involve any steps of surgery or therapy of the subject or patient.

In another alternative of the first aspect the invention provides a method of treating a patient suffering from a tumour disease, wherein the method comprises a step of administering to the patient an amount of a competitive TRXR1 binder (such as a compound of the invention), wherein the administered amount of the a competitive TRXR1 binder is determined based on the detected level of TRX1 in a tumour cell of the tumour of the patient. In this respect the amount of the competitive TRXR1 binder is increased with the amount of TRX1 determined in the diagnostic assay. On the other hand, the amount of the competitive TRXR1 binder may be decreased in a case with a reduced amount of TRX1 determined by the diagnostic of the invention.

In this aspect the invention also provides a competitive TRXR1 binder for use in a method of treatment as disclosed before. Furthermore, provided is the use of a TRXR1 binder in the manufacturing of a medicament for treating a patient suffering from a tumour disease, in accordance with the herein disclosed treatment methods.

In context of the herein disclosed invention the methods of the first aspect comprise a step of adjusting the dosing of a patient in need for a treatment with a competitive TRXR1 binder. The step of adjusting the dosing of the competitive TRXR1 binder involves that the level of TRX1 in a sample of a patient (observed TRX1 level) is compared to a control or reference level or -value of TRX1 (control TRX1 level), and wherein if the observed TRX1 level is higher (increased) compared to the control TRX1 level, the amount of competitive TRXR1 binder administered to said patient is increased compared to the amount that would have been administered to the patient if there was no significant difference between the observed and control TRX1 levels. Hence, the present invention is in part based on the fact that the higher the level of the TRX1 in the sample as determined, the higher the administered amount of the competitive TRXR1 binder will be. Similarly, in the event that the observed TRX1 level is lower than the control TRX1 level, the amount of competitive TRXR1 binder administered to said patient is also decreased or reduced compared to the amount that would have been administered to the patient if there was no significant difference between the observed- and control TRX1 levels.

In a preferred embodiment, in first steps of the method, a sample of the tumour of the patient is analysed for the level of TRX1, preferably the first steps comprising:
(a) Detecting the level of thioredoxin 1 (TRX1) in a sample from the patient;
(b) Comparing the detected level of TRX1 in the sample from the patient with a reference value or with a level of TRX1 in a reference sample.

Then, it is preferably that the subsequent treatment comprises administration of the competitive TRXR1 binder to the patient together with a cyclodextrin, preferably wherein the competitive TRXR1 binder is administered as a pharmaceutical composition comprising the competitive TRXR1 binder together with a cyclodextrin.

A disease to be treated/diagnosed or stratified with the methods of the invention is, preferably, a tumour disease selected from a chemotherapy resistant tumour disease, and/or is a small cell lung cancer (SCLC) or lymphoma.

In context of the herein disclosed invention the term "competitive TRXR1 binder" is a compound that reversibly binds to TRXR1 in competition with the TRXR1 substrate TRX1. Most preferably a competitive TRXR1 binder is a competitive TRXR1 inhibitor and therefore interferes with the biological function of TRXR1. Even more preferably the competitive TRXR1 binder is a compound that reversibly binds to TRXR1. In other words, the competitive TRXR1 binder of the invention binds to TRX1 in competition with its substrate but can also dissociate from TRXR1.

Competitive TRXR1 binders or inhibitors of the invention are preferably compounds of the structure of formula (Ia) or (I). The aspect of these compounds and their medical application is disclosed herein below.

In the following a detailed description of the components of the combination of the present invention is provided. The preferred embodiments and species of each component are to be combined in any possible way with any of the preferred embodiments and species of the other components of the inventive combination.

In the following description of the compounds of the invention, the dithiocarbamates are either compounds as disclosed previously, for example in WO 2018/069525 A1, or are improved dithiocarbamates and newly described herein. Preferably, the dithiocarbamates are provided as dimeric dithiocarbamates, which in context of the invention shall mean a structure where two dithiocarbamates form a complex with a metal, preferably as depicted in formula I or Ia of the invention. However, it is also part of the invention that dithiocarbamate compounds can be provided as monomers and separately from a metal. The latter embodiment is useful if the active dimeric dithiocarbamate metal complex is prepared either immediately before administration to a patient, or are administered to a patient separately to form the active complex in the patient after administration. Hence, in its broadest meaning the term dithiocarbamate compounds of the invention shall comprise all possible variants that are suitable to form the active dithiocarbamate metal complexes according to the invention.

A dithiocarbamate compound according to the invention has preferably the general formula: R¹R²NCS₂, wherein R¹ and R² are the same or different, and are selected from hydrogen, and unsubstituted or substituted alkyl, unsubstituted or substituted alkenyl, unsubstituted or substituted aryl, and unsubstituted or substituted heteroaryl.

Even more preferably the dithiocarbamate is a dithiocarbamate disulfide of the formula R¹R²NCS₂-S₂CNR³R⁴, wherein R¹, R², R³ and R⁴ are the same or different, and are selected from hydrogen, and unsubstituted or substituted alkyl, unsubstituted or substituted akenyl, unsubstituted or substituted aryl, and unsubstituted or substituted heteroaryl, or either R¹ with R² and/or R³ with R⁴ and the adjacent N atom together form an N-heterocyclic ring.

The dithiocarbamate disulfide is a disulfide form of a dithiocarbamate and for the purposes of the present invention is preferably selected from the group consisting of diethyldithiocarbamate, pyrrolidinedithiocarbamate, (*N*-methyl, *N*-ethyl)dithiocarbamates, imidazolinedithiocarbamates, dibenzyldithiocarbamate, dimethyldithiocarbamate, dipropyldithiocarbamate, dibutyldithiocarbamate, diamyldithiocarbamate, (*N*-methyl, *N-*cyclopropylmethyl)dithiocarbamate, bis(hydroxylethyl)-dithiocarbamate and *N-*methylglucosamine dithiocarbamate.

A preferred species of dithiocarbamate of the invention is a tetraalkyl thiuram disulfide. Most preferably the dithiocarbamate is disulfiram, which has the general formula R¹R²NCS₂-S₂CNR³R⁴, where R¹, R², R³ and R⁴ are ethyl. Disulfiram is a chemical agent also known as Antabuse® or tetraethylthiuram disulfide, and is an FDA-approved drug that is widely used for the treatment of alcoholism.

According to the present invention the dithiocarbamate is combined with cyclodextrin to yield the surprising anti-tumour activities as shown in the appended examples. The term cyclodextrin stands here for cyclic oligosaccharides formed from glucose molecules connected via α-1,4-glycoside bonds. They comprise a Greek letter as prefix, depending on the number of glucose molecules from which they are built. α-, β-, γ- and δ-cyclodextrins with 6, 7, 8 or 9 glucose molecules are especially of importance.

The cyclodextrins according to the invention also include modified cyclodextrins. Modified cyclodextrins can in particular be obtained by modifying one or more of the primary and/or secondary hydroxyl groups. Modified cyclodextrins and methods for their synthesis are well known in art. The choice between a natural or modified cyclodextrin is less of importance according to the invention. Surprisingly, it has now been found that the addition of cyclodextrin to a dithiocarbamate for the formulation of the dithiocarbamate/heavy metal composition resulted in a product with excellent and synergistic anti-tumour activity. Thus, it is up to a person skilled in the art to use either a natural or suitably modified cyclodextrin which will result in the surprising effects of the herein disclosed invention.

A preferred cyclodextrin for use in the present invention is selected from an unsubstituted or substituted α-cyclodextrin, β-cyclodextrin, γ-cyclodextrin, hydroxypropyl-β-cyclodextrin and dihydroxypropyl-β-cyclodextrin. Most preferred is dihydroxypropyl-β-cyclodextrin, sulfobutylether-β-cyclodextrin, or methyl-β-cyclodextrin.

The cyclodextrin is most preferably selected from any of the following commercially available cyclodextrins:
alpha-cyclodextrin (CAS #: 10016-20-3)
alpha-cyclodextrin phosphate Sodium salt (CAS #: 199684-60-1)
alpha-cyclodextrin, sulfated Sodium salt Hydrate (CAS #: 699020-02-5)
Hexakis (2,3,6-tri-O-acetyl)-alpha-cyclodextrin
Hexakis (2,3,6-tri-O-methyl)-alpha-cyclodextrin
Hexakis(2,3,6-tri-O-octyl)-alpha-cyclodextrin (CAS #: 140395-31-9)
Hexakis-6-bromo-6-deoxy-alpha-cyclodextrin (CAS #: 53784-82-0)
Hexakis-6-iodo-6-deoxy-alpha-cyclodextrin (CAS #: 131105-41-4)
Hexakis (6-O-tertbutyl-dimethylsilyl)-alpha-cyclodextrin
Butyl-alpha-cyclodextrin
Succinyl-alpha-cyclodextrin
(2-Hydroxypropyl)-alpha-cyclodextrin (CAS #: 128446-33-3)
beta-cyclodextrin (CAS #: 7585-39-9)
beta-cyclodextrin Hydrate (CAS #: 68168-23-0)
beta-cyclodextrin phosphate Sodium salt (CAS #: 199684-61-2)
beta-cyclodextrin sulfate
beta-cyclodextrin, sulfated Sodium salt (CAS #: 37191-69-8)
Hydroxypropyl-beta-cyclodextrin (CAS #: 94035-02-6)
6-Monodeoxy-6-monoamino-beta-cyclodextrin
6-O-alpha-D-Glucosyl-beta-cyclodextrin (CAS #: 92517-02-7)
6-O-alpha-Maltosyl-beta-cyclodextrin Hydrate (CAS #: 104723-60-6) Heptakis-6-azido-6-deoxy-beta-cyclodextrin
Heptakis(2,3-di-O-acetyl-6-O-sulfo)-beta-cyclodextrin Heptasodium salt (CAS #: 196398-66-0)
Heptakis-(2,3-di-O-methyl-6-O-sulfo)-beta-cyclodextrin Heptasodium salt (CAS #: 201346-23-8)
Heptakis(2,6-di-O-methyl)-beta-cyclodextrin (CAS #: 51166-71-3)
Heptakis-(2,6-di-O-ethyl)-beta-cyclodextrin (CAS #: 111689-03-3)
Heptakis(2,3,6-tri-O-methyl)-beta-cyclodextrin (CAS #: 55216-11-0)
Heptakis(2,3,6-tri-O-acetyl)-beta-cyclodextrin
Heptakis-(2,3,6-tri-O-benzoyl)-beta-cyclodextrin (CAS #: 23666-43-5)
Heptakis-(2,3,6-tri-O-ethyl)-beta-cyclodextrin (CAS #: 111689-01-1)
Heptakis-6-iodo-6-deoxy-beta-cyclodextrin (CAS #: 30754-23-5)
Heptakis-6-(dimethyl-tert-butylsilyl)-6-deoxy-beta-cyclodextrin
Heptakis-6-bromo-6-deoxy-beta-cyclodextrin
Monoacetyl-beta-cyclodextrin
Diacetyl-beta-cyclodextrin
Triacetyl-beta-cyclodextrin (CAS #: 23739-88-0)
Heptakis(3-O-acetyl-2,6-di-O-methyl)-beta-cyclodextrin (CAS #: 131889-29-7)
Heptakis-(6-O-maltosyl)-beta-cyclodextrin
Heptakis(6-O-sulfo)-beta-cyclodextrin Heptasodium salt (CAS #: 197587-31-8)
Heptakis(6-O-t-butyldimethylsilyl-2,3-di-O-acetyl)-beta-cyclodextrin
Succinyl-(2-hydroxypropyl)-beta-cyclodextrin
(2,6-Di-O-)ethyl-beta-cyclodextrin
(2-Carboxyethyl)-beta-cyclodextrin
(2-Hydroxyethyl)-beta-cyclodextrin (CAS #: 128446-32-2)
(2-Hydroxypropyl)-beta-cyclodextrin (CAS #: 128446-35-5)
Butyl-beta-cyclodextrin
Methyl-beta-cyclodextrin (CAS #: 128446-36-6)
Silyl((6-O-tert-butyldimethyl)-2,3,-di-O-acetyl)-beta-cyclodextrin
Succinyl-beta-cyclodextrin
gamma-cyclodextrin (CAS #: 17465-86-0)
gamma-cyclodextrin Hydrate (CAS #: 91464-90-3)
gamma-cyclodextrin phosphate Sodium salt (CAS #: 199684-62-3)
Sulfopropyl-beta-cyclodextrin
Carboxymethyl-gamma-cyclodextrin
Octakis (2,3,6-tri-O-acetyl)-gamma-cyclodextrin
Octakis (2,3,6-tri-O-methyl)-gamma-cyclodextrin
Octakis (2,6-di-O-pentyl)-gamma-cyclodextrin
Octakis-6-(dimethyl-tert-butylsilyl)-6-deoxy-gamma-cyclodextrin
Octakis-6-bromo-6-deoxy-gamma-cyclodextrin (CAS #: 53784-84-2)
Octakis-6-iodo-6-deoxy-gamma-cyclodextrin (CAS #: 168296-33-1)
Octakis (6-O-t-butyldimethylsilyl)-gamma-cyclodextrin
Succinyl-gamma-cyclodextrin
(2-Hydroxypropyl)-gamma-cyclodextrin (CAS #: 128446-34-4)
Acetyl-gamma-cyclodextrin
Butyl-gamma-cyclodextrin
Betadex Sulfobutyl Ether

One of the particularly preferred cyclodextrins of the invention is hydroxypropyl-beta-cyclodextrin (CAS #: 94035-02-6) or methyl-beta-cyclodextrin (CAS #: 128446-36-6) or SBE-β-CD|Sulfobutylether-β-cyclodextrin(CAS #: 182410-00-0).

The "heavy metal source" or "heavy metal ion source" shall refer to any compound providing the heavy metal atom for the formation of a complex of the heavy metal and the dithiocarbamate of the invention. Particularly preferred heavy metals are selected from arsenic, bismuth, cobalt, copper, chromium, gallium, gold, iron, manganese, nickel, platin, silver, titanium, vanadium, selenium, and zinc; and preferably the source is a copper or more preferably gold source.

In some embodiments the heavy metal ion source is a chelate of a heavy metal ion and a sulfate salt, a chloride salt or an organic anion, such as acetate, lactate, glycinate, citrate, propionate, and gluconate. As mentioned already above, in some embodiments the source of the heavy metal according to the invention is a dithiocarbamate-metal complex. In this embodiment in particular complexes of gold formed with disulfiram is preferred. Metal complexes of dithiocarbamates are known for example from Hogarth G.: "Metal-dithiocarbamate complexes: chemistry and biological activity." (Mini Rev Med Chem. 2012 Oct;12(12):1202-15. Review. PubMed PMID: 22931592; incorporated by reference in its entirety), in particular the structures of figure 9. A preferred complex has the following dimeric dithiocarbamate-metal structure Ia:

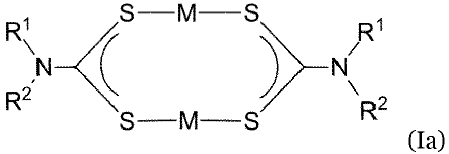

Wherein R¹ and R² are the same or different, and are selected from hydrogen, and unsubstituted or substituted alkyl, unsubstituted or substituted akenyl, unsubstituted or substituted aryl, unsubstituted or substituted alkoxy, and unsubstituted or substituted heteroaryl; and preferably are ethyl; and wherein M is a metal ion.

In some preferred embodiments the compounds of the invention (the complexes) are in polymeric form - so-called catena-complexes - due to intermolecular metal-metal interactions.

In some aspects the present invention further pertains to the following complexes of formula (Ia), wherein the R¹ and R² are the same or different, and are selected from hydrogen, and unsubstituted or substituted C₁ to C₂₀, preferably C₁ to C₅ alkyl, unsubstituted or substituted C₁ to C₂₀, preferably C₁ to C₅ akenyl, unsubstituted or substituted aryl, unsubstituted or substituted alkoxy, and unsubstituted or substituted heteroaryl; and preferably are ethyl; and wherein M is a metal ion selected from Au or Cu.

An alkyl or alkenyl may be straight, branched or cyclic in context of the invention.

The term "substituted", as used herein, in relation to the above moieties refers to a substituent other than hydrogen. Such a substituent is preferably selected from the group consisting of halogen, - CF₃, -C₂F₅, -C₃F₇, -C₄F₉, -C₅F₁₁, and other fluoroalkyl of 2 to 5 carbons, -OH, -NH₂, -NO₂, -CHO, -CN, -COOH, -SH, -SO₂OH, -CONH₂, -NHNH₂, -OR, -NRR',-C(O)R, -C(O)OR, -(CO)NRR', -NR'C(O)R, -OC(O)R, aryl with 5 to 10 carbon atoms, cycloalk(en)yl with 3 to 20 carbon atoms, 3- to 8-membered heterocycloalk(en)yl, and 5- to 10-membered heteroaryl, wherein R and R' are independently selected from hydrogen, alkyl with 1 to 10 carbon atoms, alkenyl with 2 to 10 carbon atoms, alkynyl with 2 to 10 carbon atoms, aryl with 5 to 14 carbon atoms, cycloalk(en)yl with 3 to 20 carbon atoms, 5- to 14-membered heteroaryl, comprising 1 to 4 heteroatoms selected from nitrogen, oxygen, and sulfur, and 5- to 14-membered heterocycloalk(en)yl, comprising 1 to 4 heteroatoms selected from nitrogen, oxygen, and sulfur. In some embodiments any of these substituents may again be substituted, it is however preferred that these substituents are unsubstituted.

In some embodiments R¹ and R² may be connected to form a 4, 5, 6, 7 or 8-membered *N-*heterocyclic ring system, which is optionally substituted.

The term "alkyl", as used herein, refers to a saturated hydrocarbon moiety, such as methyl, ethyl, and the like. The terms "alkenyl" and "alkynyl", as used herein, comprise aliphatic residues with at least one carbon-carbon double bond or triple bond, respectively, and are otherwise defined as "alkyl".

The term "cycloalkyl", as used herein, refers to a non-aromatic carbocyclic moiety, such as cyclopentanyl, cyclohexanyl, and the like. The term "cycloalkenyl", as used herein, refers to non-aromatic carbocyclic compounds that comprise at least one carbon-carbon double bond.

Similarly, the term "heterocycloalk(en)yl" as used herein, relates to cycloalk(en)yl groups wherein 1 or more ring carbon atoms are replaced by heteroatoms, preferably selected from nitrogen, oxygen and sulfur.

The term "aryl", as used herein, relates to an aromatic ring that is preferably monocyclic or consists of condensed aromatic rings. Preferred aryl substituents are moieties with 6 to 14 carbon atoms, such as phenyl, naphthyl, anthracenyl, pyridinyl and phenanthrenyl.

The term "heteroaryl" as used herein, refers to aromatic moieties that correspond to the respective aryl moiety wherein one or more ring carbon atoms have been replaced by heteroatoms, such as nitrogen, sulfur, oxygen, phosphorus. Preferred heteroaryls are pyrrolyl, imidazolyl, furanyl and thiophenyl and the like.

As used herein, the term "metal ion" refers to elements of the periodic table that are metallic and that are, preferably, positively charged. Metals that are useful in the present invention include the earth metals, alkali earth metals, transition metals and post-transition metals. Alkali metals include Li, Na, K, Rb and Cs. Alkaline earth metals include Be, Mg, Ca, Sr and Ba. Transition metals include Sc, Ti, V, Cr, Mn, Fe, Co, Ni, Cu, Zn, Y, Zr, Nb, Mo, Tc, Ru, Rh, Pd, Ag, Cd, La, Hf, Ta, W, Re, Os, Ir, Pt, Au, Hg and Ac. Post-transition metals include Al, Ga, In, Tl, Ge, Sn, Pb, Sb, Bi, and Po. One of skill in the art will appreciate that the metals described above can each adopt several different oxidation states, all of which are useful in the present invention. Preferred metals are Au and Cu.

Preferred complexes according to the invention have any one of the following structures: 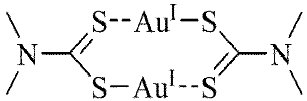 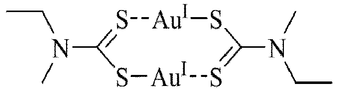 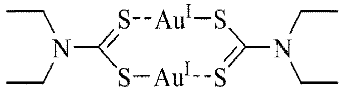 (The above compound is also referred to as "DKFZ-608")

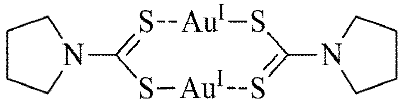

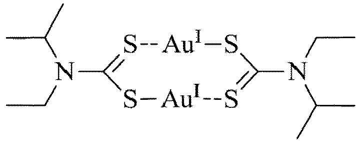

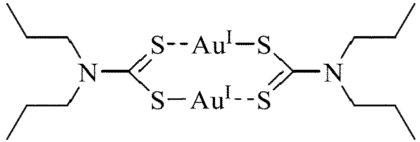

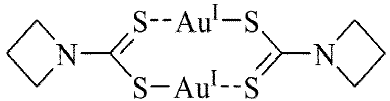

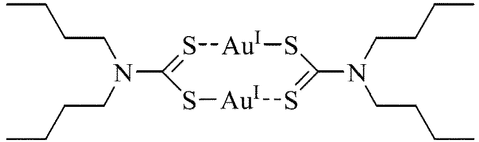

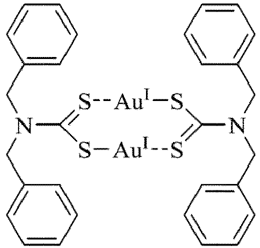

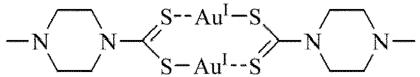

A compound for use in a treatment is preferred, wherein the treatment comprises administration of the compound together with a cyclodextrin, preferably wherein the compound is administered as a pharmaceutical composition comprising the compound together with a cyclodextrin. The cyclodextrin is preferably selected as stated elsewhere herein.

Another aspect then pertains also to a pharmaceutical composition for use in the treatment of a disease, comprising a compound or complex as disclosed herein, together with a cyclodextrin and a pharmaceutical acceptable carrier and/or excipient.

In another aspect the invention provides any one the following compounds:

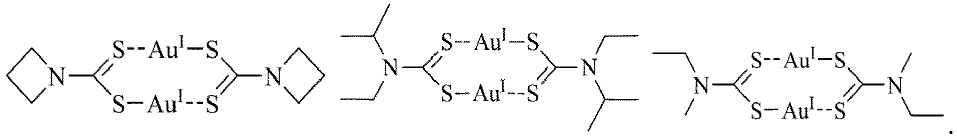

The term "chemotherapy-resistant tumour" as used herein refers to a tumour disease, including the individual cells therein, that is or becomes refractory to treatment by a chemotherapy, such as the therapy with a chemotherapeutic agent, for example etoposide or platin compounds. In specific embodiments, the chemotherapy-resistant tumour becomes resistant after initiation of the treatment and may occur during the treatment. In further specific embodiments, the resistance to chemotherapy manifests at about 2-24 months while the patient is receiving chemotherapy. In *de novo* resistance, the patient does not respond to initial therapy. Acquired resistance is where the patient develops metastatic disease during therapy. Acquired resistance to chemotherapy is well-known in the art. In particular, small cell lung cancer patients while undergoing treatment with chemotherapy have recurrence of the disease, usually resulting in the death of the patient. In specific embodiments, the disease metastasizes during therapy with anthracycline based chemotherapy, which results in resistant metastases.

Preferred metal sources of the invention may be selected from auranofin, aurothiomalate, Au-dithiocarbamate, aurothioglucose, which are preferred sources for a metal that can be administered to a patient or used for an immediate preparation of the medicament of the invention without extensive additional purification. However, the invention shall not be restricted to any particular source of metal ions, which are well known to the skilled artisan and can be selected depending on the purpose.

According to the invention a preferred combination pertains to the combination of a disulfiram, a copper or gold compound, and a cyclodextrin as described herein above.

In a preferred embodiment of the invention a combination is preferred wherein the dithiocarbamate, the source of a heavy metal and the cyclodextrin are in admixture in the combination and not spatially separated.

In some embodiments it might be preferred that the combination of the invention further comprises at least one additional cytotoxic compound. In cancer therapy it is often useful to combine cancer therapeutics with different modes of anti-cancer activity to avoid the development of resistant forms of the disease. The at least one additional cytotoxic compound may be selected from cyclophosphamide ifosfamide, hexamethylmelamine, tirapazimine, sertenef, cachectin, tasonermin, lonidamine, carboplatin, mitomycin, altretamine, prednimustine, dibromodulcitol, ranimustine, fotemustine, nedaplatin, oxaliplatin, temozolomide, doxorubicin, heptaplatin, estramustine, improsulfan tosilate, trofosfamide, nimustine, dibrospidium chloride, pumitepa, lobaplatin, satraplatin, profiromycin, cisplatin, irofulven, dexifosfamide, *cis*-aminedichloro(2-methyl-pyridine) platinum, benzylguanine, glufosfamide, GPX100, diarizidinylspermine, arsenic trioxide, zorubicin, idarubicin, daunorubicin, bisantrene, mitoxantrone, pirarubicin, pinafide, valrubicin, amrubicin, antineoplaston, annamycin, galarubicin, elinafide, MEN10755, and 4-demethoxy-3'-deamino-3'-aziridinyl-4-methylsulphonyldaunorubicin, rapamycin and its derivatives, sirolimus, temsirolimus, everolimus, zotarolimus and deforolimus. Also included in the definition are microtubulin inhibitors like paclitaxel, vindesine sulfate, 3',4'-didehydro-4'-deoxy-8'- norvincaleukoblastine, docetaxel, rhizoxin, dolastatin, mivobulin isethionate, auristatin, cemadotin, RPR109881, BMS 184476, vinflunine, cryptophycin, 2,3,4,5,6-pentafluoro-*N*-(-3-fluoro-4-methoxyphenyl)benzene sulfonamide, anhydrovinblastine, *N,N-*dimethyl-*L*-valyl-*L*-valyl-*N*-methyl-*L*-valyl-*L*-prolyl-*L*-proline-*t*-butylamide, TDX258, BMS 188797, topotecan, hycaptamine, irinotecan, rubitecan, 7-[2-(N-isopropyl amino)ethyl]-(20S)camptothecin, BNP1350, BNPI1100, BN80915, BN80942, etoposide phosphate, teniposide, sobuzoxane, 2'-dimethylamino-2'-deoxyetoposide, GL331, N-[2-(dimethylamino)ethyl]-9-hydroxy-5,6-dimethyl-6H-pyrido[4,3-b]carbazole-1-carboxamide, asulacrine, 6,9-bis[(2-amino-ethyl)amino]benzo[g]isoquinoline-5, 10-dione, N-[1-[2(diethylamino)ethylamino]-7-methoxy-9-oxo-9H-thioxanthen-4-ylmethyl]formamide, *N-*(2-(dimethylamino)ethyl)acridine-4-carboxamide, and dimesna.

Preferred for the combination with disulfiram are in particular carboplatin, oxaliplatin, cisplatin and/or etoposide, vinorelbine, or similar compounds.

In preferred embodiments of the invention the combination comprises cyclodextrin in amounts higher than the amount of the dithiocarbamate, or dithiocarbamate/metal complex respectively. Therefore, the invention pertains to inventive combination wherein the (molar) ratio of cyclodextrin:dithiocarbamate, or cyclodextrin:dithiocarbamate/metal, ranges from at least 0.01 (1:100) to 500 (500:1), or preferably 0.1 to 50. More preferred are ratios of 2, 5, 10, 50, 100, and most preferred is a ratio of about 500 +/- 50.

The combination of the invention may include the components in the form of a kit, either in separate or admixed form. The components in the kit could be provided in liquid form or in solid form, for example to allow for a resuspension or solution of the components in a pharmaceutically acceptable liquid right before use. The kit of the invention may in some embodiments comprise the source of the heavy metal separately from the dithiocarbamate (not admixed with the dithiocarbamate).

### Improved Dimeric Dithiocarbamate Compounds:

A second aspect of the present invention then provides a compound selected from a (dimeric) dithiocarbamate metal complex according to the following formula (I), or any solvates, salts, stereoisomers, polymorphs, crystalline forms, racemic mixtures, diastereomers, enantiomers, tautomers, isotopically labelled forms, prodrugs, and combinations thereof 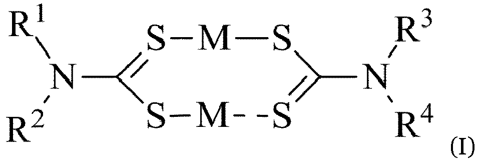
wherein R¹, R², R³ and 4 are the same or different, or where R¹ and R² and/or R³ and R⁴ are connected to form a 3- to 8-membered ring, and are selected from hydrogen, and unsubstituted or substituted C₁ to C₂₀ straight, branched or cyclic alkyl, unsubstituted or substituted C₁ to C₂₀ straight, branched or cyclic alkenyl, unsubstituted or substituted C₁ to C₂₀ alkynyl, unsubstituted or substituted aryl, and unsubstituted or substituted heteroaryl; and
wherein M is a metal ion;
**characterized in that**
R¹, R², R³ and/or R⁴ comprise at least one free hydroxyl group (-OH group).

As used herein, the term free hydroxyl group refers to the functional group (-OH). In some embodiments, the surface hydroxyl group may be present in the form of an organic acid, such as a carboxyl group, or in the form of an alcohol such as a -OH group. The free hydroxyl group of the invention is preferably attached to an aliphatic or aromatic carbon atom. In other embodiments the at least one free hydroxyl group is attached to a hetero atom, preferably selected from nitrogen, oxygen, sulfur and phosphor.

In further preferred embodiments R¹ and R² do not form a ring; and R¹ comprises at least one free hydroxyl group, and R² does not comprise a free hydroxyl group, or wherein R² comprises at least one free hydroxyl group, and R₁ does not comprise a free hydroxyl group.

Furthermore, R¹ and R² may form a 3- to 10-membered ring, and wherein said ring comprises at least one free hydroxyl group.

The compound of the invention is preferred, wherein, if R¹ and R² do not form a ring, R¹ and/or R² each comprise not more than one free hydroxyl group; or wherein, if R¹ and R² do form a 3- to 10-membered ring, the ring of R¹ and R² together comprise not more than one free hydroxyl group.

In yet another preferred embodiment the 3- to 10-membered ring is optionally substituted with another heteroatom such as S, O and/or N.

For example, the at least one free hydroxyl group is provided in context of any one of the following groups:

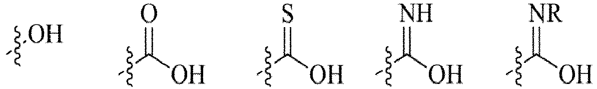

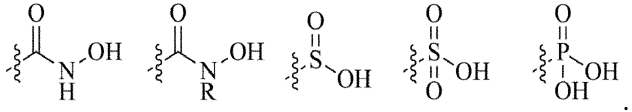

A substituent for a compound of the invention may be selected from halogen, -CF₃, -C₂F₅,-C₃F₇, -C₄F₉, -C₅F₁₁ and other fluoroalkyl of 2 to 5 carbon atoms, -OH, -NH₂, -NO₂, -CHO, -CN, -COOH, -CONHOH, -SH, SO₂OH, -CONH₂, -NHNH₂, -OR, -NRR', -C(O)R, -C(O)OR,-C(O)NRR', -NR'C(O)R, -OC(O)R, aryl ring with 5 to 10 carbon atoms, cycloalk(en)yl rings with 3 to 20 carbon atoms, 3- to 10-membered heterocycloalk(en)yl rings, and 5- to 10-membered heteroaryl rings, wherein R and R' are independently selected from hydrogen, straight or branched alkyl chains with 1 to 10 carbon atoms, straight or branched alkenyl chains with 2 to 10 carbon atoms, straight or branched alkynyl chains with 2 to 10 carbon atoms, aryl rings with 5 to 14 carbon atoms, cycloalk(en)yl rings with 3 to 20 carbon atoms, 5- to 14-membered heteroaryl rings, comprising 1 to 4 heteroatoms selected from nitrogen, oxygen, sulfur and phosphor, and 5- to 14-membered heteroalk(en)yl rings, comprising 1 to 4 heteroatoms selected from nitrogen, oxygen, sulfur and phosphor, and optionally wherein said substituent is further substituted.

The compound of the invention may be in the form of a polymer, preferably a catena complex. However, in some embodiments the compound of the invention has any one of the following structures:

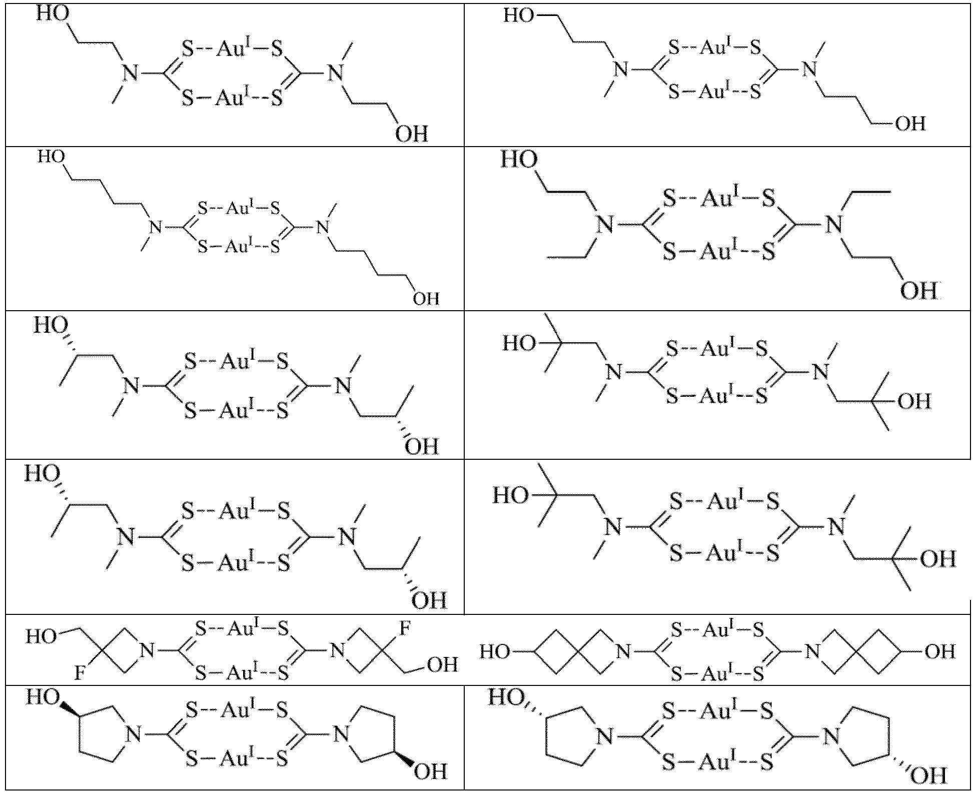

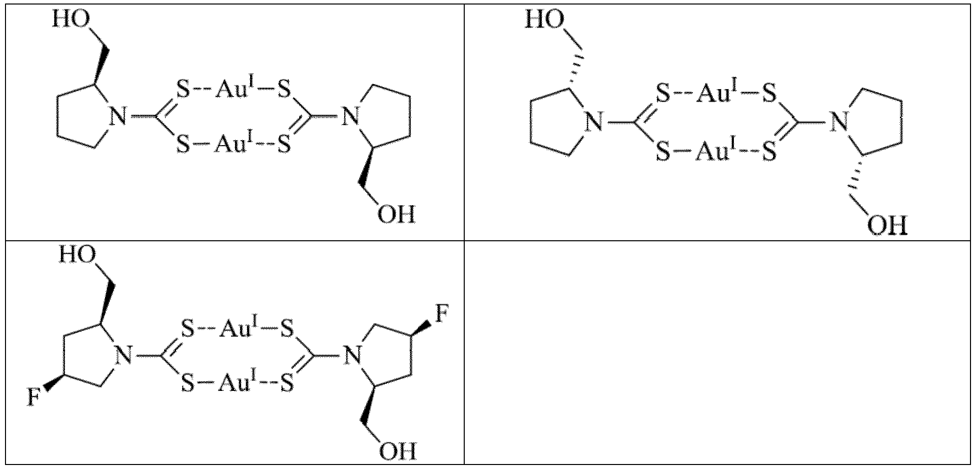

It may be preferred that R¹ and/or R² is covalently connected to R³ and/or R⁴, and wherein the covalent connection comprises a linker molecule, preferably wherein the linker comprises an unsubstituted or substituted C₁ to C₂₀ straight, branched or cyclic alkyl, unsubstituted or substituted C₁ to C₂₀ straight, branched or cyclic alkenyl, unsubstituted or substituted C₁ to C₂₀ alkynyl, and/or unsubstituted or substituted aryl, and unsubstituted or substituted heteroaryl.

The herein disclosed advantageous compounds according to formula (I) can be used as dithiocarbamate compounds in context of a diagnostic method of the herein disclosed first aspect.

Within this second aspect, furthermore provided is a method for producing a small molecular anti-cancer agent with increased *in vivo* anti-cancer activity, wherein a candidate small molecular anti-cancer agent is provided that does not comprise a free hydroxyl group, and wherein the method comprises a step of modifying the candidate small molecular anti-cancer agent to include at least one free hydroxyl group to obtain a small molecular anti-cancer agent with increased *in vivo* anti-cancer activity. For example, the step of modifying the candidate small molecular anti-cancer agent to include at least one free hydroxyl group is comprised in the method of chemically synthesizing the candidate small molecular anti-cancer agent, or involves enzymatically modifying the candidate small molecular anti-cancer agent, for example using a CYP enzyme.

As already mentioned before, the term "cancer" or "tumour" in some instances refers to a solid cancer, preferably SCLC.

The method according to any one of claims 37 to 39, wherein the structure of the candidate small molecular anti-cancer agent differs from the structure of the obtained small molecular anti-cancer agent with increased *in vivo* anti-cancer activity only in the presence of the free hydroxyl group.

For example a candidate small molecular anti-cancer agent is preferably a dithiocarbamate, for example any of the compounds recited for the first aspect, and thus preferably a compound as defined in WO 2018/069525 A1.

The invention further provides a compound obtained by a method described herein above.

Another embodiment of the second aspect then pertains to a pharmaceutical composition comprising the compound of the second aspect, and pharmaceutically acceptable carrier and/or excipient, optionally wherein the carrier and/or excipient includes cyclodextrin.

A pharmaceutical composition of the invention may be in the form of solid components, a lyophilized solution, an injectable solution, or a solid composition (lyophilized composition of the single or combined components of the combination of the invention) for the preparation of an injectable solution, a capsule, a tablet, a cream, an ointment, or an oral or nasal inhalation composition.

As used herein, the term "pharmaceutical composition" refers to the combination of an active agent with a carrier, inert or active, making the composition especially suitable for diagnostic or therapeutic use in vivo, in vivo or ex vivo.

As used herein, the term "pharmaceutically acceptable carrier" refers to any of the standard pharmaceutical carriers, such as a phosphate buffered saline solution, water, emulsions (e.g., such as an oil/water or water/oil emulsions), and various types of wetting agents. The compositions also can include stabilizers and preservatives. For examples of carriers, stabilizers and adjuvants, see e.g., Martin, Remington's Pharmaceutical Sciences, 15th Ed., Mack Publ. Co., Easton, PA [1975].

As used herein, the term "pharmaceutically acceptable salt" refers to any pharmaceutically acceptable salt (e.g., acid or base) of a compound of the present invention which, upon administration to a subject, is capable of providing a compound of this invention or an active metabolite or residue thereof. As is known to those of skill in the art, "salts" of the compounds of the present invention may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their pharmaceutically acceptable acid addition salts.

For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

In context of the here disclosed first and second aspect of the invention, medical uses of the dithiocarbamates are contemplated.

Therefore, the invention also provides a product for use in the treatment of a disease, wherein the product is selected from any of the herein disclosed dithiocarbamate compounds or the pharmaceutical composition comprising the same. Hence, the invention also provides methods of treating such herein indicated disease by administration of the products of the invention to a subject in need of the treatment. Alternative embodiments describe the use of the products of the invention in the manufacture of a medicament for treating any of the herein indicated diseases.

Such a disease of the invention is preferably a proliferative disease, preferably a cancer disease. However, other disease may also be treated with the compounds of the invention, as will be apparent from the herein elsewhere described third aspect of the invention.

A treatment which is in accordance with the invention preferably comprises administration of a dithiocarbamate compound of the invention, together with a cyclodextrin, preferably wherein the compound is administered as a pharmaceutical composition, comprising the compound together with a cyclodextrin.

Generally, a tumour or cancer disease that is susceptible to the treatment of the invention is a a tumour disease, such as a tumour selected from a chemotherapy resistant tumour disease, and/or is a SCLC, preferably wherein the tumour disease is a chemotherapy resistant, and/or relapsed SCLC. Preferably, cells of a tumour disease according to the invention are characterized by a reduced expression of TRX1, or by a lower expression of TRX1 compared to a reference cell. A reference cell may be selected from a cell which is derived from a tumour that is not efficiently treatable with the dithiocarbamate compound according to the invention, or alternatively the reference cell is a healthy cell.

Further provided is a combination, comprising as components: a dithiocarbamate intermediate of the herein disclosed compounds (the dithiocarbamates), a cyclodextrin and a heavy metal source. An intermediate is preferably a dithiocarbamate compound used together with a metal source to form the metal-dithiocarbamate compounds disclosed herein.

As mentioned a cyclodextrin is preferably selected from an unsubstituted or substituted α-cyclodextrin, β-cyclodextrin, γ-cyclodextrin, hydroxypropyl-β-cyclodextrin and dihydroxypropyl-β-cyclodextrin, preferably is dihydroxypropyl-β-cyclodextrin, sulfobutylether-β-cyclodextrin or methyl-β-cyclodextrin.

A heavy metal source is preferably selected from a source of arsenic, bismuth, cobalt, copper, chromium, gallium, gold, iron, manganese, nickel, platinum, silver, titanium, vanadium, selenium, and zinc; and preferably is a copper or gold source, such as auranofin, aurothiomalate, aurothioglucose, Au-dithiocarbamate, or alternatively are gold(I) chloride, chloro(dimethylsulfide)gold(I), hydrogen tetrachloroaurate(III), sodium/potassium gold(III)chloride, copper(I/II)chloride and copper(II)sulfate.

The combination of the invention for example may include embodiments wherein the heavy metal source is administered to the subject separately from the dithiocarbamate intermediate. Alternatively, the dithiocarbamate and the cyclodextrin are administered as one composition simultaneously.

The medical uses of the herein disclosed aspects of the invention preferably involve the following:
The compounds of the present invention are useful in the preparation of medicaments to treat any of the above disorders. The methods and techniques for preparing medicaments of a compound of the invention are well-known in the art. Exemplary pharmaceutical formulations and routes of delivery are described herein elsewhere.

One of skill in the art will appreciate that any one or more of the compounds described herein, including the many specific embodiments, are prepared by applying standard pharmaceutical manufacturing procedures. Such medicaments can be delivered to the subject by using delivery methods that are well-known in the pharmaceutical arts.

Most preferably the disease to be treated according to the invention is cancer. A cancer according to the invention may be selected from a solid or liquid tumour disease, preferably selected from fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumour, leiomyosarcoma, rhabdomyosarcoma, colon carcinoma, colorectal cancer, anal carcinoma, esophageal cancer, gastric cancer, hepatocellular cancer, bladder cancer, endometrial cancer, pancreatic cancer, brain cancer, breast cancer, ovarian cancer, prostate cancer, stomach cancer, atrial myxomas, squamous cell carcinoma, basal cell carcinoma, adenocarcinoma, sweat gland carcinoma, sebaceous gland carcinoma, thyroid and parathyroid neoplasms, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumour, cervical cancer, testicular tumour, lung carcinoma, small cell lung carcinoma, non-small-cell lung cancer, bladder carcinoma, epithelial carcinoma, glioma, pituitary neoplasms, astrocytoma, medulloblastoma, craniopharyngioma, ependymoma, pinealoma, hemangioblastoma, acoustic neuroma, schwannomas, oligodendroglioma, meningioma, spinal cord tumours, melanoma, neuroblastoma, pheochromocytoma, Types 1-3 endocrine neoplasia, retinoblastoma; leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenström's macroglobulinemia, and heavy chain disease.

A preferred group of tumour diseases to be treated with the combinations, compounds and compositions of the invention is selected from lung cancer, colon carcinoma, ovary cancer, liver cancer, mamma carcinoma, pancreatic cancer, melanoma, glioma, T-cell lymphoma, leukemia, and Burkitt lymphoma.

In two particular preferred embodiments the cancer to be treated or prevented in context of the herein disclosed invention is SCLC (most preferred) or alternatively lymphoma. As supported by the appended examples, the compounds and compositions of the invention are surprisingly effective in the treatment of SCLC for which effective treatment strategies are desperately needed. Also lymphoma was surprisingly well treatable with the compounds and compositions of the invention.

A preferred embodiment of the invention pertains to the use of the combination or compounds for use in the treatment of a chemotherapy resistant tumour disease, for example a tumour disease characterized by the expression of an ABC transporter protein such as MDR1 or other transport proteins such as MRP1, MXR, BCRP or ABC-P. Many mechanisms of cancer chemotherapy resistance have been discovered to date including other mechanisms of reduced compound uptake by the cancer cells such as by a reduced endocytosis or pinocytosis or by inhibition of apoptosis. The present invention shall not be restricted to any particular mechanism of chemoresistance. The surprising synergism between the dithiocarbamate/metal complex and cyclodextrin allows overcoming chemotherapy resistance in tumour disorders and therefore provides new therapeutic strategies for patients in advanced disease stages. Therefore, the tumour disease is in some embodiments a refractory tumour disease.

Although many routes for administration of the combination or compounds of the invention are included by this invention, a preferred embodiment relates to formulations of the combinations of the invention which are suitable for parenteral administration. Such formulations include aqueous and non-aqueous isotonic sterile injection solutions which may contain antioxidants, buffers, bacteriostats and solutes which render the formulation isotonic with the blood of the intended recipient; and aqueous and non-aqueous sterile suspensions which may include suspending agents and thickening agents, and liposomes or other microparticulate systems which are designed to target the compound to blood components or one or more organs. In some embodiments, the formulations are presented/formulated in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily subdose, as herein above -recited, or an appropriate fraction thereof, of an agent.

Various delivery systems are known and can be used to administer a therapeutic agent (e.g., a combination of disulfiram/metal ion and cyclodextrin according to the invention), e.g., encapsulation in liposomes, micro particles, microcapsules, receptor-mediated endocytosis, and the like. Methods of delivery include, but are not limited to, intra-arterial, intramuscular, intravenous, intranasal, and oral routes. In specific embodiments, it may be desirable to administer the pharmaceutical compositions of the invention locally to the area in need of treatment; this may be achieved by, for example, and not by way of limitation, local infusion during surgery, injection, or by means of a catheter.

The combination and compounds of the invention for the purposes of treatment can be administered to subjects or individuals diagnosed with for example a cancer disease. When the agent is administered to a subject such as a mouse, a rat or a human patient, the agent can be added to a pharmaceutically acceptable carrier and systemically or topically administered to the subject.

In some embodiments, *in vivo* administration is effected in one dose, continuously or intermittently throughout the course of treatment. Methods of determining the most effective means and dosage of administration are well known to those of skill in the art and vary with the composition used for therapy, the purpose of the therapy, the target cell being treated, and the subject being treated. Single or multiple administrations are carried out with the dose level and pattern being selected by the treating physician.

Suitable dosage formulations and methods of administering the agents are readily determined by those of skill in the art. Preferably, the compounds are administered at about 0.01 mg/kg to about 500 mg/kg, more preferably at about 0.1 mg/kg to about 100 mg/kg, even more preferably at about 0.5 mg/kg to about 50 mg/kg. When the compounds described herein are co-administered with another agent (e.g., as sensitizing agents), the effective amount may be less than when the agent is used alone.

More particularly, an agent of the present invention also referred to herein as the active ingredient, may be administered for therapy by any suitable route including, but not limited to, oral, rectal, nasal, topical (including, but not limited to, transdermal, aerosol, buccal and sublingual), vaginal, parental (including, but not limited to, subcutaneous, intramuscular, intravenous and intradermal) and pulmonary. It is also appreciated that the preferred route varies with the condition and age of the recipient, and the disease being treated.

Ideally, the agent should be administered to achieve peak concentrations of the active compound at sites of disease. This may be achieved, for example, by the intravenous injection of the agent, optionally in saline, or orally administered, for example, as a tablet, capsule or syrup containing the active ingredient. Desirable blood levels of the agent may be maintained by a continuous infusion to provide a therapeutic amount of the active ingredient within disease tissue. The use of operative combinations is contemplated to provide therapeutic combinations requiring a lower total dosage of each component than may be required when each individual therapeutic compound or drug is used alone, thereby reducing adverse effects.

The compounds of the invention or components of the combination are administered either to the subject separately, or one component separately, and the other two combined, or all combined. For example, in some embodiment it might be preferred that the source of a metal ion is administered to the subject separately from the dithiocarbamate and the cyclodextrin. In other embodiments the dithiocarbamate, the metal source and the cyclodextrin are administered as one composition simultaneously.

In addition the present invention further concerns the following specifically preferred items:
Item 1: A method for diagnosing a tumour disease in a patient, the method comprising the steps of
   (a) Detecting the level of thioredoxin 1 (TRX1) in a sample from the patient;
   (b) Comparing the detected level of TRX1 in the sample from the patient with a reference value or with a level of TRX1 in a reference sample,
   wherein a reduced level of TRX1 in the sample from the patient compared to the reference value or with the level of TRX1 in a reference sample indicates a tumour which is treatable with a competitive thioredoxin reductase 1 (TRXR1) inhibitor.
Item 2: A method for deciding how to treat a tumour disease in a patient, the method comprising the steps of
   (c) Detecting the level of thioredoxin 1 (TRX1) in a sample from the patient;
   (d) Comparing the detected level of TRX1 in the sample from the patient with a reference value or with a level of TRX1 in a reference sample,
   wherein a reduced level of TRX1 in the sample from the patient compared to the reference value or with the level of TRX1 in a reference sample indicates a treatment of the patient with a competitive thioredoxin reductase 1 (TRXR1) inhibitor.
Item 3: The method according to item 1 or 2, wherein the sample is a biological sample and obtained from the patient, preferably wherein the biological sample comprises at least one tumour cell of the tumour disease to be diagnosed.
Item 4: The method according to item 3, wherein the biological sample is a liquid sample or a tissue sample, such as a sample obtained from a tumour biopsy or tumour resection.
Item 5: The method according to any one of items 1 to 4, wherein detecting the level of TRX1 in the sample involves detection of the level of protein and/or mRNA of TRX1, and/or the TRX1 promoter region methylation status.
Item 6: The method according to item 5, wherein the methylation in the promoter region is detected at CpG sites at positions -995, and /or -1418 distal to the translational start site (ATG) in the TRX1 gene, or to a region not more than 100bp, preferably 50bp, 40bp, or 20bp, preferably 10bp, most preferably 5bp away from positions -995, and /or -1418.
Item 7: The method according to item 5 or 6, wherein the TRX1 promoter region comprises at least one response element for oxidative stress.
Item 8: The method according to any one of items 1 to 7, wherein the patient is a human, and wherein the TRX1 is human TRX1, and wherein the TRXR1 is human TRXR1.
Item 9: The method according to any one of items 1 to 8, wherein the method is an in vitro or ex vivo method, or wherein the method does not involve any steps of surgery or therapy of the patient.
Item 10: A method of treating a patient suffering from a tumour disease, wherein the method comprises a step of administering to the patient an amount of a competitive TRXR1 binder, wherein the administered amount of the a competitive TRXR1 binder is calculated based on the detected level of TRX1 in a tumour cell of the tumour of the patient.
Item 11: The method according to item 10, wherein the higher the level of the TRX1 in the sample, the higher is the administered amount of the competitive TRXR1 binder.
Item 12: The method according to item 10 or 11, wherein in first steps a sample of the tumour of the patient is analyzed for the level of TRX1, preferably the first steps comprising:
   (e) Detecting the level of thioredoxin 1 (TRX1) in a sample from the patient;
   (f) Comparing the detected level of TRX1 in the sample from the patient with a reference value or with a level of TRX1 in a reference sample.
Item 13: The method according to any one of items 10 to 12, wherein the treatment comprises administration of the competitive TRXR1 binder to the patient together with a cyclodextrin, preferably wherein the competitive TRXR1 binder is administered as a pharmaceutical composition comprising the competitive TRXR1 binder together with a cyclodextrin.
Item 14: The method according to any one of items 10 to 13, wherein the disease is a tumour disease selected from a chemotherapy resistant tumour disease, and/or is a small cell lung cancer (SCLC) or lymphoma.
Item 15: The method according to any one of the preceding items, wherein the competitive TRXR1 binder is a competitive TRXR1 inhibitor, and preferably is a compound that reversibly binds to TRXR1.
Item 16: The method according to any one of the preceding items, wherein the competitive TRXR1 binder binds reversibly and competitively with TRX1 to TRXR1.
Item 17: The method according to any one of the preceding items, wherein the competitive TRXR1 binder is a compound being selected from a complex according to the following formula (Ia): 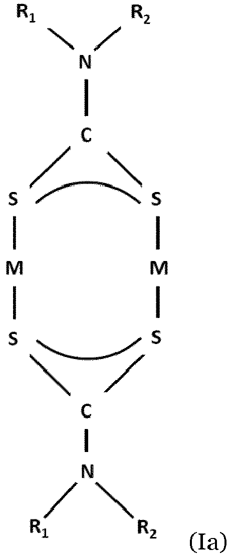
   wherein the R₁ and R₂ are the same or different, or are connected to form a N-heterocyclic 3-to 6-membered ring, and are selected from hydrogen, and unsubstituted or substituted C₁ to C₁₀ straight, branched or cyclic alkyl, unsubstituted or substituted C₁ to C₁₀ straight, branched or cyclic akenyl, unsubstituted or substituted aryl, and unsubstituted or substituted heteroaryl; and wherein M is a metal ion;
   or a diastereomer, enantiomer or a pharmaceutical acceptable salt thereof.
Item 18: The method according to any one of the preceding items, wherein the tumour is selected from a chemotherapy resistant tumour disease, and/or is a small cell lung cancer (SCLC) or lymphoma. a lung cancer, preferably is small cell lung cancer (SCLC).
Item 19: The method according to item 15 or 16, wherein the competitive TRXR1 binder is a compound being selected from any one of the following compounds: 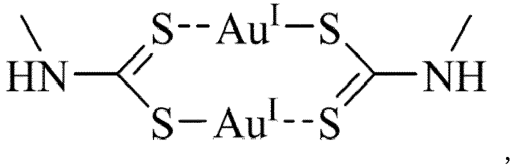 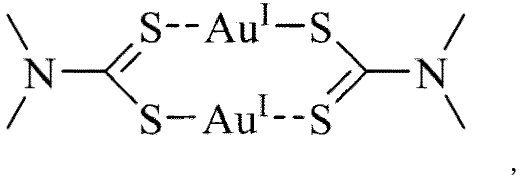 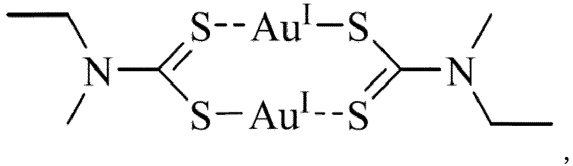 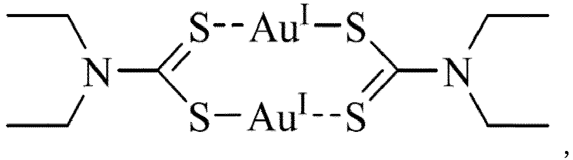 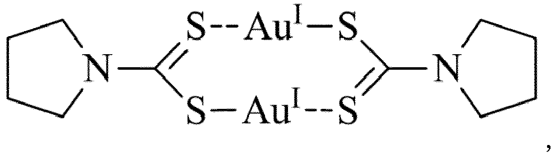 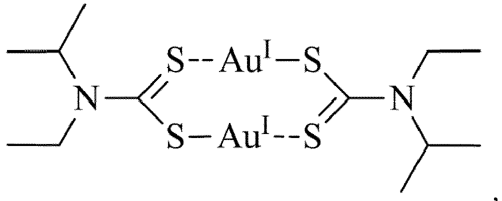 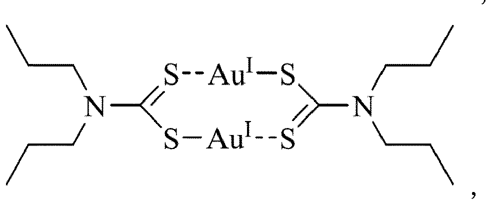 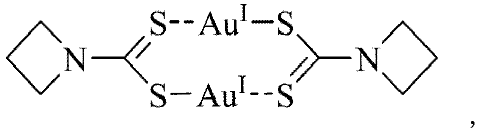 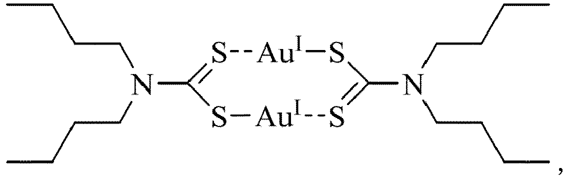 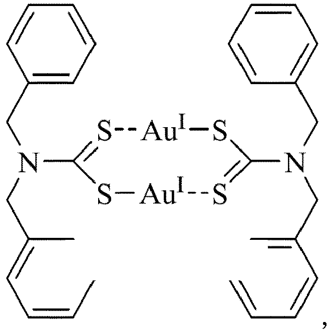 and 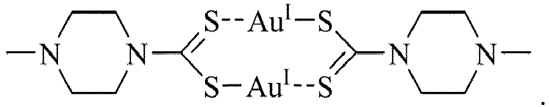
Item 20: The method according to any one of the preceding items, wherein the competitive TRXR1 binder is a compound having the following structure: 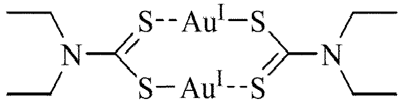
Item 21: A competitive TRXR1 binder for use in the treatment of a tumour disease, wherein the treatment involves a method according to any one of items 1 to 20.
Item 22: A compound selected from a dimeric dithiocarbamate metal complex according to the following formula (I), or any solvates, salts, stereoisomers, polymorphs, crystalline forms, racemic mixtures, diastereomers, enantiomers, tautomers, isotopically labelled forms, prodrugs, and combinations thereof: 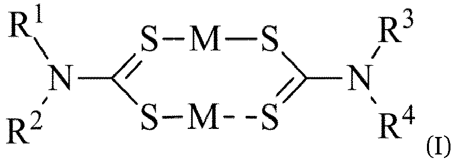
   wherein R¹, R², R³ and 4 are the same or different, or where R¹ and R² and/or R³ and R⁴ a connected to form a 3- to 8-membered ring, and are selected from hydrogen, and unsubstituted or substituted C₁ to C₁₀ straight, branched or cyclic alkyl, unsubstituted or substituted C₁ to C₁₀ straight, branched or cyclic alkenyl, unsubstituted or substituted C₁ to C₁₀ alkynyl, unsubstituted or substituted aryl, and unsubstituted or substituted heteroaryl; and
   wherein M is a metal ion;
   **characterized in that**
   R¹, R², R³ and/or R⁴ comprise at least one free hydroxyl group (-OH group).
Item 23: The compound according to item 22, wherein the at least one free hydroxyl group is attached to an aliphatic or aromatic carbon atom.
Item 24: The compound according to item 22, wherein the at least one free hydroxyl group is attached to a hetero atom, preferably selected from nitrogen, oxygen, sulfur and phosphorous.
Item 25: The compound according to any one of items 22 to 24, wherein R¹ and R² do not form a ring; and wherein R¹ comprises at least one free hydroxyl group, and R² does not comprise a free hydroxyl group, or wherein R² comprises at least one free hydroxyl group, and R₁ does not comprise a free hydroxyl group.
Item 26: The compound according to any one of items 22 to 24, wherein R¹ and R² form a 3- to 10-membered ring, and wherein said ring comprises at least one free hydroxyl group.
Item 27: The compound according to any one of items 22 to 26, wherein, if R¹ and R² do not form a ring, R¹ and/or R² each comprise not more than one free hydroxyl group; or wherein, if R¹ and R² do form a 3- to 10-membered ring, the ring of R¹ and R² together comprise not more than one free hydroxyl group.
Item 28: The compound according to any one of the preceding items, wherein the 3- to 10-membered ring, optionally substituted with another heteroatom such as S, O and/or N.
Item 29: The compound according to any one of the preceding items, wherein the at least one free hydroxyl group is provided in context of the any of the following groups: 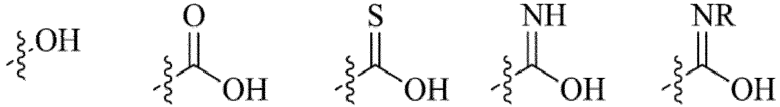 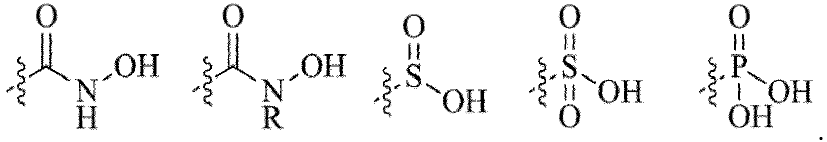
Item 30: The compound according to any one of the preceding items, wherein a substituent is selected from halogen, -CF₃, -C₂F₅, -C₃F₇, -C₄F₉, -C₅F₁₁ and other fluoroalkyl of 2 to 5 carbon atoms, -OH, -NH₂, -NO₂, -CHO, -CN, -COOH, -CONHOH, -SH, SO₂OH, -CONH₂, -NHNH2, -OR, -NRR', -C(O)R, -C(O)OR, -C(O)NRR', -NR'C(O)R, -OC(O)R, aryl ring with 5 to 10 carbon atoms, cycloalk(en)yl rings with 3 to 20 carbon atoms, 3- to 10-membered heterocycloalk(en)yl rings, and 5- to 10-membered heteroaryl rings, wherein R and R' are independently selected from hydrogen, straight or branched alkyl chains with 1 to 10 carbon atoms, straight or branched alkenyl chains with 2 to 10 carbon atoms, straight or branched alkynyl chains with 2 to 10 carbon atoms, aryl rings with 5 to 14 carbon atoms, cycloalk(en)yl rings with 3 to 20 carbon atoms, 5- to 14-membered heteroaryl rings, comprising 1 to 4 heteroatoms selected from nitrogen, oxygen, sulfur and phosphor, and 5- to 14-membered heteroalk(en)yl rings, comprising 1 to 4 heteroatoms selected from nitrogen, oxygen, sulfur and phosphorous, and optionally wherein said substituent is further substituted.
Item 31: The compound according to any one of items 22 to 30, in the form of a polymer, preferably a catena complex.
Item 32: The compound according to any one of the preceding items having any one of the the following structures: 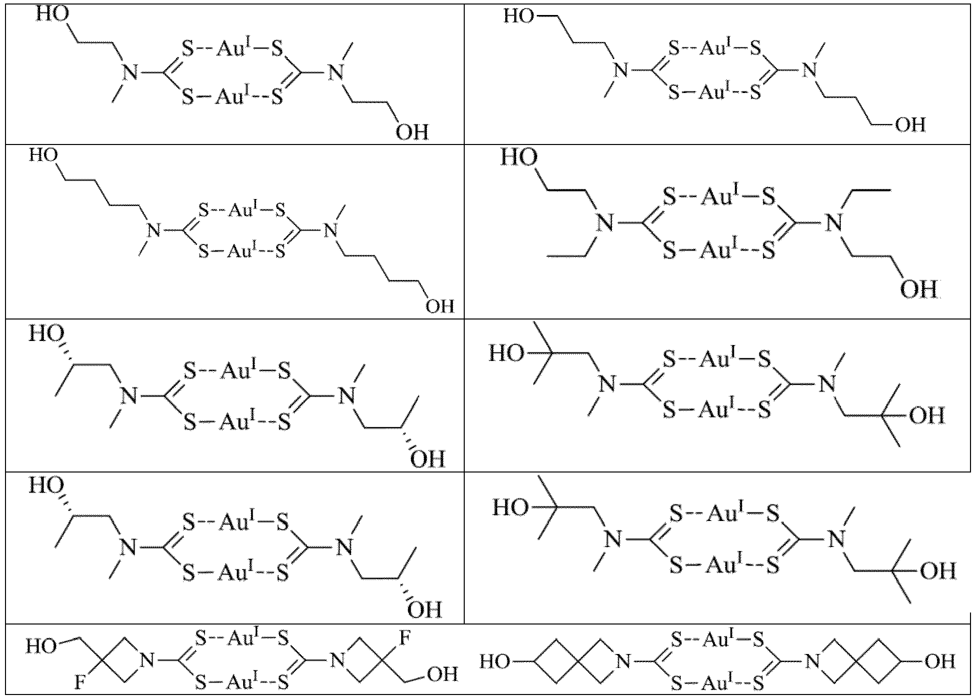 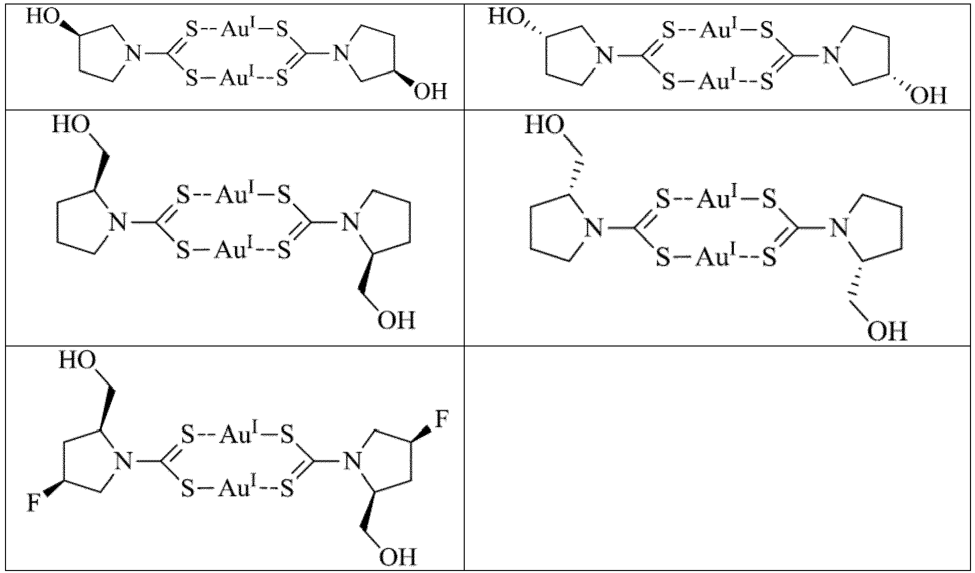
Item 33: The compound according to any one of items 22 to 30, wherein R¹ and/or R² is covalently connected to R³ and/or R⁴, and wherein the covalent connection comprises a linker molecule, preferably wherein the linker comprises an unsubstituted or substituted C₁ to C₂₀ straight, branched or cyclic alkyl, unsubstituted or substituted C₁ to C₂₀ straight, branched or cyclic alkenyl, unsubstituted or substituted C₁ to C₂₀ alkynyl, and/or unsubstituted or substituted aryl, and unsubstituted or substituted heteroaryl.
Item 34: A method according to any one of items 1 to 16, wherein the competitive TRXR1 inhibitor is a compound according to any one of items 22 to 35.
Item 35: A method for producing a small molecular anti-cancer agent with increased *in vivo* anti-cancer activity, wherein a candidate small molecular anti-cancer agent is provided that does not comprise a free hydroxyl group, and wherein the method comprises a step of modifying the candidate small molecular anti-cancer agent to include at least one free hydroxyl group to obtain a small molecular anti-cancer agent with increased *in vivo* anti-cancer activity.
Item 36: The method according to item 37, wherein the step of modifying the candidate small molecular anti-cancer agent to include at least one free hydroxyl group is comprised in the method of chemically synthesizing the candidate small molecular anti-cancer agent, or involves enzymatically modifying the candidate small molecular anti-cancer agent, for example using a CYP enzyme.
Item 37: The method according to item 37 or 38, wherein the cancer is a solid cancer, preferably SCLC.
Item 38: The method according to any one of items 37 to 39, wherein the structure of the candidate small molecular anti-cancer agent differs from the structure of the obtained small molecular anti-cancer agent with increased *in vivo* anti-cancer activity only in the presence of the free hydroxyl group.
Item 39: The method according to any one of items 37 to 40, wherein the candidate small molecular anti-cancer agent is a dithiocarbamate, preferably a compound as defined in WO 2018/069525 A1.
Item 40: A compound obtained by a method according to any one of items 37 to 41.
Item 41: A pharmaceutical composition comprising the compound of any one of items 22 to 35, or 42, and pharmaceutically acceptable carrier and/or excipient, optionally wherein the carrier and/or excipient includes cyclodextrin.
Item 42: A product for use in the treatment of a disease, wherein the product is selected from a compound of any one of items 22 to 35, or 42, or the pharmaceutical composition of item 21.
Item 43: The product for use according to item 44, for use in a treatment of a proliferative disease, preferably a cancer disease.
Item 44: The product for use according to item 45, wherein the cancer is a solid tumour, and preferably is SCLC.
Item 45: The product for use according to any one of items 44 to 46, wherein the treatment comprises administration of a compound according to any one of items 22 to 35, or 42, together with a cyclodextrin, preferably wherein the compound is administered as a pharmaceutical composition, preferably a pharmaceutical composition according to item 43, comprising the compound together with a cyclodextrin.
Item 46: The product for use according to item 47, and any preceding items when referring to item 46, wherein the disease is a tumour disease, preferably TRX positive tumour disease, selected from a chemotherapy resistant tumour disease, and/or is a SCLC, preferably wherein the tumour disease is a chemotherapy resistant, and/or relapsed SCLC.
Item 47: A combination comprising the components: a dithiocarbamate intermediate of the compound according to any one of items 22 to 35, a cyclodextrin and a heavy metal source.
Item 48: The combination according to item 49, wherein the cyclodextrin is selected from an unsubstituted or substituted α-cyclodextrin, β-cyclodextrin, γ-cyclodextrin, hydroxypropyl-β-cyclodextrin and dihydroxypropyl-β-cyclodextrin, preferably is dihydroxypropyl-β-cyclodextrin, sulfobutylether-β-cyclodextrin or methyl-β-cyclodextrin.
Item 49: The combination according to item 49 or 50, wherein the heavy metal source is selected from a source of arsenic, bismuth, cobalt, copper, chromium, gallium, gold, iron, manganese, nickel, platinum, silver, titanium, vanadium, selenium, and zinc; and preferably is a copper or gold source, such as auranofin, aurothiomalate, aurothioglucose, Au-dithiocarbamate, copper(I/II)chloride and copper(II)sulfate.
Item 50: The combination according to any one of items 49 to 51, wherein the dithiocarbamate intermediate and the heavy metal source are provided as the compound according to any one of items 22 to 35.
Item 51: The combination according to any one of items 49 to 52, further comprising at least one additional cytotoxic compound.
Item 52: The combination according to any one of items 49 to 53, wherein the components of the combination are a combined or separate pharmaceutical composition(s), and wherein the pharmaceutical composition(s) further comprise(s) a pharmaceutically acceptable carrier and/or excipient.
Item 53: The combination according to any one of items 49 to 54, for use in the treatment of a tumour disease, rheumatic arthritis, or persisting HIV infection.
Item 54: The combination for use according to item 55, wherein the tumour disease is selected from lung cancer, most preferably is SCLC, colon carcinoma, ovary cancer, liver cancer, mamma carcinoma, pancreatic cancer, melanoma, glioma, T-cell lymphoma, leukemia, and Burkitt lymphoma.
Item 55: The combination for use according to item 49 or 50, wherein the heavy metal source is administered to the subject separately from the dithiocarbamate intermediate. Item 56: The combination for use according to any one of items 49 to 57, wherein the dithiocarbamate and the cyclodextrin are administered as one composition simultaneously.

The present invention will now be further described in the following examples with reference to the accompanying figures and sequences, nevertheless, without being limited thereto. For the purposes of the present invention, all references as cited herein are incorporated by reference in their entireties. In the example section either a "," or "." is used as decimal mark. In the Figures:

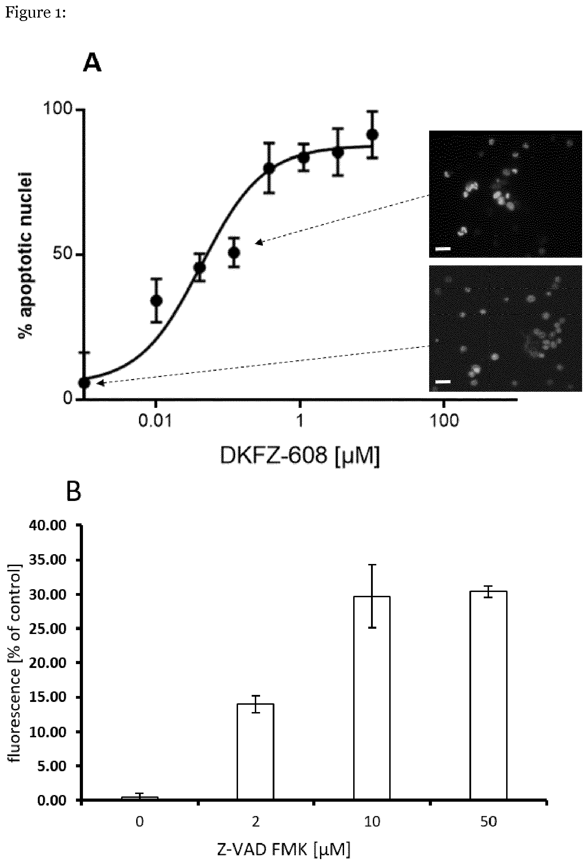

- **Figure 1:**: Induction of apoptotic cell death by DKFZ-608. **A:** Induction of chromatin condensation by DKFZ-608. Scale bar indicates 20 µm. Photos show examples treated with o and 120 nM DKFZ-608. **B:** DKFZ-608-induced cytotoxicity in H209 is partially rescued by the pan caspase inhibitor Z-VAD FMK. Cytoprotection is calculated as % fluorescence signal relative to the untreated control.

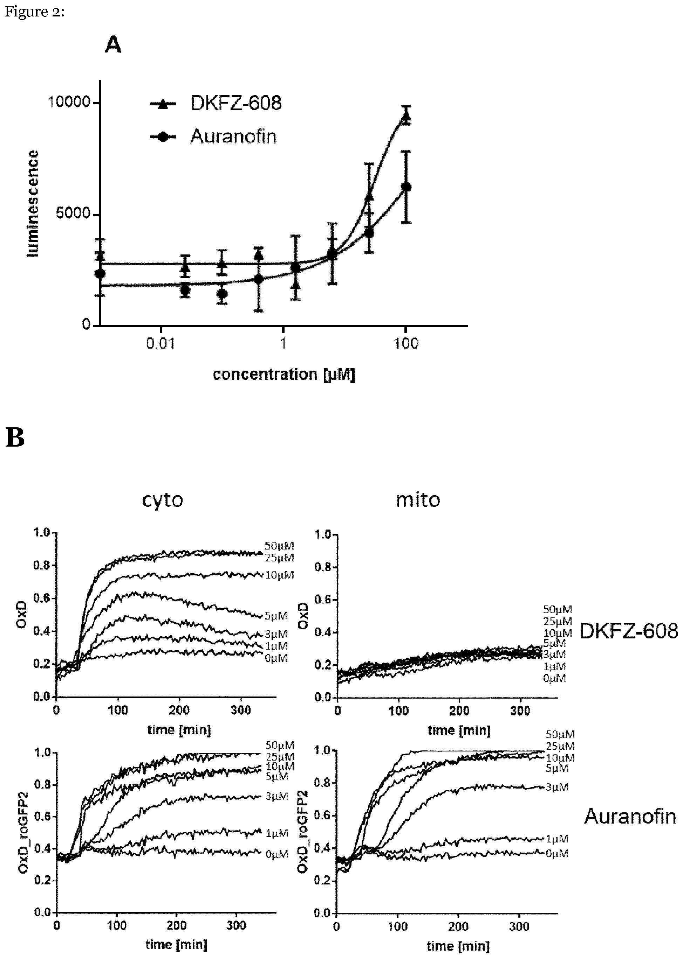

- **Figure 2**:: **A:** DKFZ-608 induces an increase in intracellular H₂O₂. Mean values +/- SD were plotted. **B:** Time course of roGFP2-Orp1 oxidation induced by DKFZ-608 or auranofin.

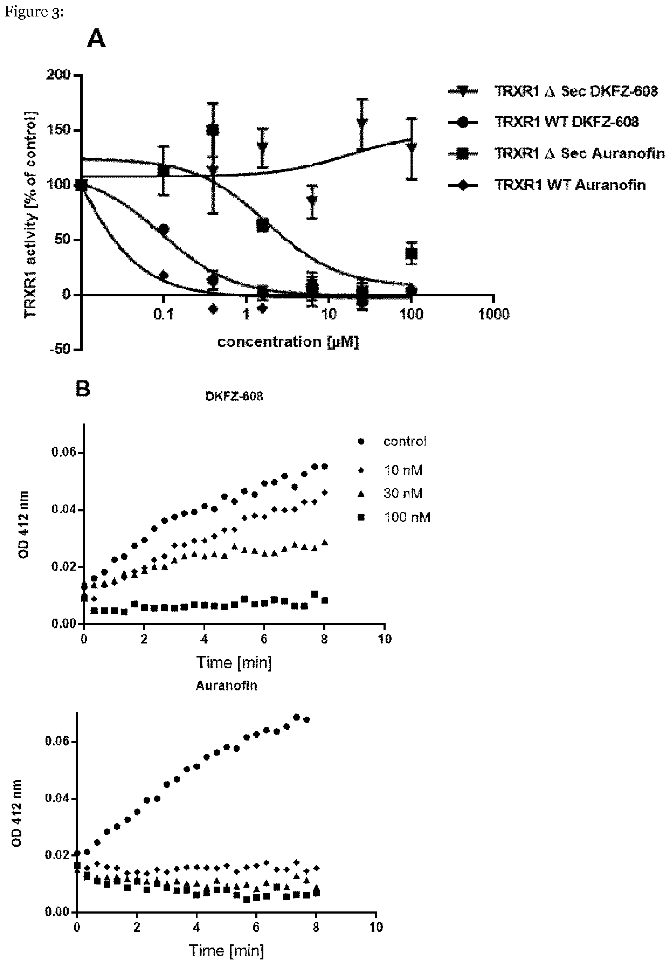

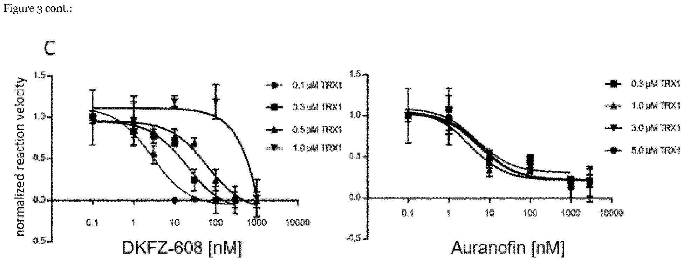

- **Figure 3:**: **A:** DKFZ-608 mode of inhibition requires the C-terminal selenocysteine. The effect of DKFZ-608 and auranofin on the activity of cloned human wild type and Sec-deficient TRXR1 was determined with DTNB. **B:** DKFZ-608, in contrast to auranofin, dissociates from TRXR1 upon dilution leading to recovery of enzyme activity. **C:** TRX1, the natural substrate of TRXR1, affects enzyme inhibition by DKFZ-608 Inhibitors were pre-incubated with TRXR1 before the addition of TRX1.

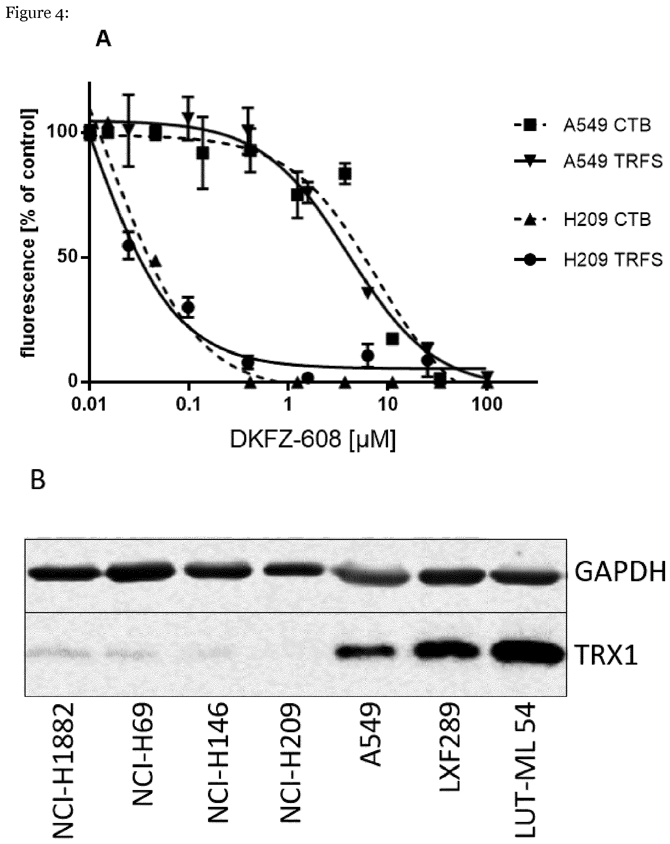

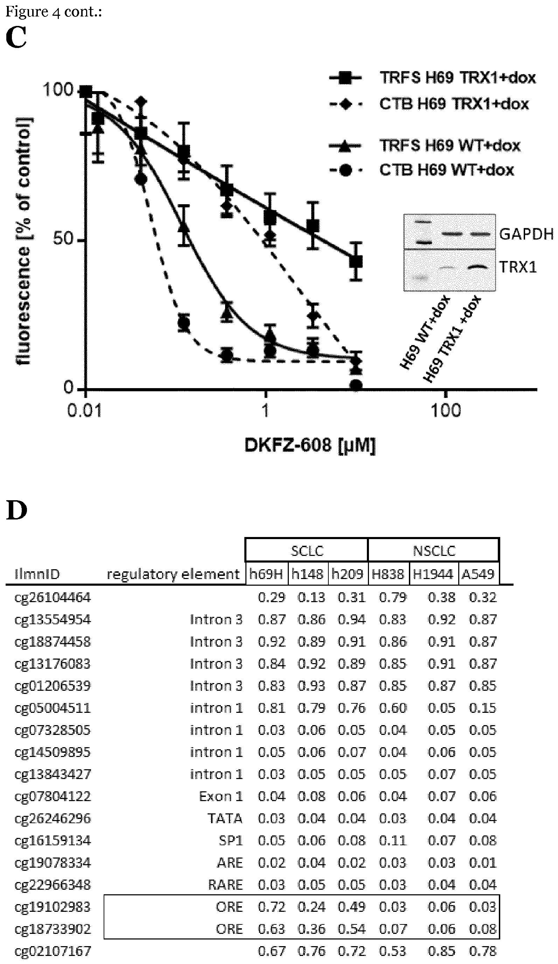

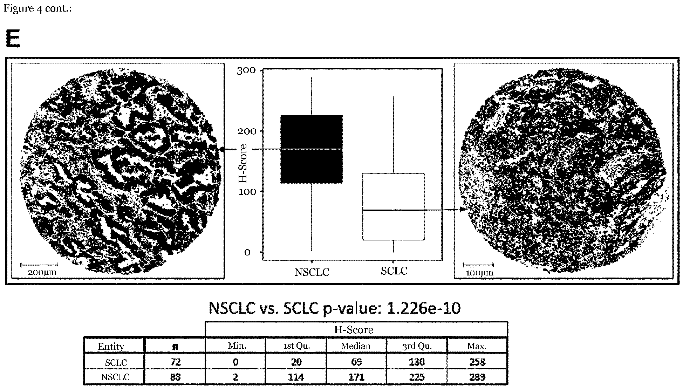

- **Figure 4:**: **A:** Dose dependent inhibition of TRXR1 and cytotoxicity are highly correlated in lung cancer cells. Assessment of cytotoxicity after 72h and of TRXR1 inhibition after 100min. All data were normalized (untreated control = 1). **B:** SCLC cell lines have lower intrinsic levels of TRX1 as compared to NSCLC cell lines. **C:** Overexpression of TRX1 in H69 cells leads to a reduction of TRXR1 inhibition and a desensitization of cells to DKFZ-608. Assessment of cytotoxicity after 72h and of TRXR1 inhibition after 100min. All data were normalized (untreated control = 100%). **D**: Low expression levels of TRX correlate with hypermethylation of a distal promoter region containing an oxidative stress response element. The heat map depicts beta values with o= no methylation and 1 indicating high methylation status. CpG position relative to start codon. i.g.r.: intergenic region, ORE: oxidative stress response element, RARE: retinoic acid response element, ARE: antioxidant responsive element, SP1:SP1 binding site, Ex 1: Exon 1. **E**: Tumour samples from SCLC patients express significantly lower levels of TRX1 as compared to samples derived from NSCLC. Tumour specimen representative for the median H-score of each group are depicted.

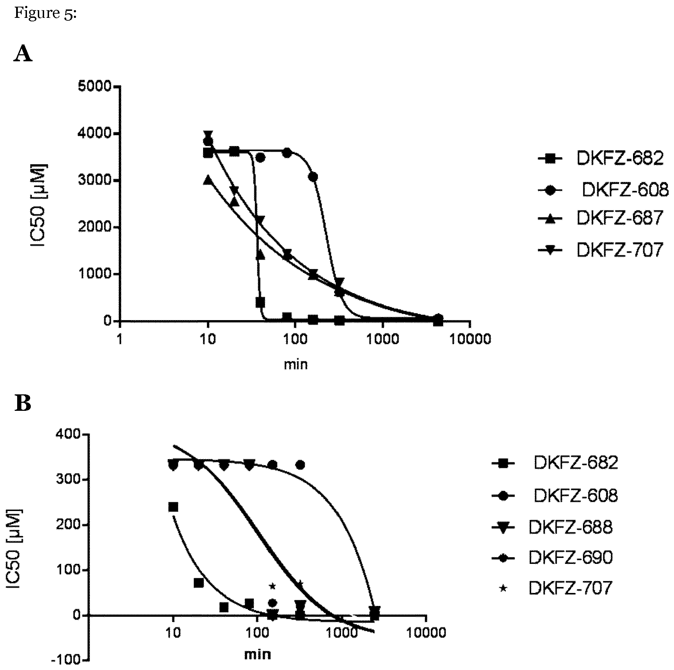

- **Figure 5:**: **A:** Effect of incubation time on antitumoral activity in A549 lung cancer cells. 4 Compounds are tested, provided is the IC50 versus treatment time. **B:** Effect of incubation time on antitumoral activity in colon cancer cells. Provided are 5 compounds and their IC50 vs. incubation time.

### EXAMPLES

Structures tested in context of the examples:

| | |
|---|---|
| 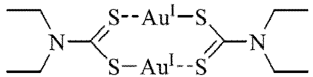 | DKFZ-608 |
| 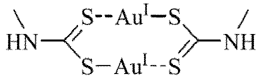 | DKFZ-616 |
| 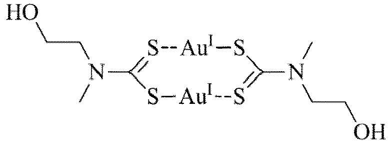 | DKFZ-680 |
| 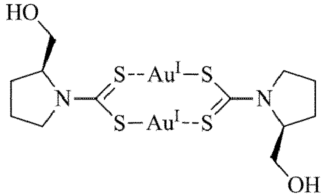 | DKFZ-682 |
| 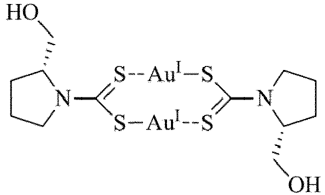 | DKFZ-687 |
| 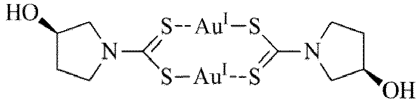 | DKFZ-688 |
| 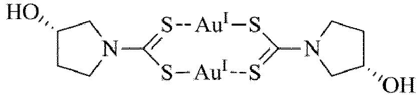 | DKFZ-690 |
| 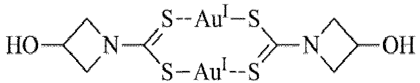 | DKFZ-696 |
| 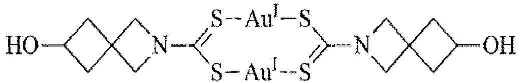 | DKFZ-707 |

### A: TRX1/TRXR1 - as a diagnostic and drug target of cancer

### Example A1: Cell death mechanisms of DKFZ-608 a previously discovered new anti cancer drug.

The compound DKFZ-608 and related compounds were identified as a particularly preferable anti-cancer drug in WO 2018/069525 A1. As an example of the new class of compounds DKFZ-608 is used in the following experiments. However, the present invention shall not be necessarily restricted to this compound:

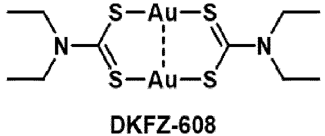

Treatment with DKFZ-608 caused morphological changes of the nucleus reminiscent of apoptosis (Figure 1A). The majority of untreated control cells displayed evenly stained, round nuclei with soft edges while, in cells treated with DKFZ-608, the nuclei stained more intensely because of chromatin condensation and showed signs of nuclear fragmentation and apoptotic bodies in a dose-dependent manner. The inventors then investigated the possible involvement of apoptotic mechanisms in the SCLC cell line H209. The cytotoxic effect of DKFZ-608 on H209 cells could be reverted by the pan-Caspase inhibitor z-VAD-FMK, indicating that caspase dependent apoptosis is an important mechanism (Figure 3B). In agreement with this, DKFZ-608 increases the amount of Annexin-V positive cells and late apoptotic cells (data not shown). It was also investigated whether alternative cell death mechanisms like ferroptosis, necroptosis or entosis contribute to the cytotoxicity of DKFZ-608 on SCLC. Inhibitors of those cell death programs were, however, not able to desensitize SCLC cells.

### Example A2: DKFZ 608 induces oxidation of redox sensitive proteins

Gold(I) complexes have been previously reported to induce pro-oxidative effects in tumour cells. We used a selection of oxidant sensing chemical and genetically-encoded probes as well as biomarkers to assess the dynamics and quality of oxidants potentially induced by DKFZ-608 and auranofin, which we used as a reference substance. Using a probe which allows to quantify peroxides (ROS GlowTM, a boronate-caged luciferin precursor we observed that the two gold complexes in that DKFZ-608 and auranofin induced fast and strong increase of peroxides (most likely H2O2) (Figure 2A).

Treatment of cells with DKFZ-608 induced formation of disulfide-linked dimers of the thiol peroxidases peroxiredoxin 1 (PRDX1) and 3 (PRDX3) as well as oxidation of TRX1 (not shown). In an orthogonal assay, which quantifies the redox state of the genetically encoded H₂O₂ probe roGFP2-Orp1 (Gutscher et al., 2009) the inventors detected concentration-dependent probe oxidation, which reached its maximum after about 60 min and remained at high levels, indicating that the machinery required to reduce the probe was blocked by the action of the drug. Two major differences between the effects caused by DKFZ-608 and auranofin were noticed: when using the mitochondrial roGFP2-Orp1 probe, auranofin produces the same fast and pronounced increase in probe oxidation as in the cytoplasm. In contrast, DKFZ-608 did not cause oxidation of the mitochondrial probe (Figure 2B). Secondly, at concentrations below the EC₅₀ of DKFZ-608 (19.48 µM ± 5 µM for H838) probe oxidation appears to be reversible while it remains stable in cells treated with the same concentrations of auranofin.

### Example A3: DKFZ-608 is a potent, competitive inhibitor of TRXR1

A likely explanation for the observed increase in H₂O₂ levels and the concomitant accumulation of oxidized PRDX1, PRDX3 and TRX1 is inhibition of the NADPH-dependent thiol reducing machinery, the TRXR1/TRX1 system being the main player next to the glutathione system. DKFZ-608 was found to inhibit the enzymatic activity of wild-type TRXR1 (Wr-TRXR1), with an IC50 that is similar to auranofin. Interestingly, DKFZ-608 appears to interact predominantly with the redox-active C-terminal selenocysteine (Sec) of WT-TRXR1 since a truncated form of TRXR1, lacking the Sec (TRXR1ΔSec), is not inhibited by DKFZ-608 (Figure 3A). In contrast, auranofin is able to repress the activity of the truncated enzyme, although to a lesser extent, probably through interactions with cysteine residues involved in the enzymatic reaction (Lothrop et al., 2009).

Surprisingly, DKFZ-608 showed characteristics incompatible with irreversible inhibition, a common mechanism of action for gold-containing substances, and auranofin in particular: while dilution of a sample containing TRXR1 and a high concentration of DKFZ-608 resulted in recovery of enzymatic activity, this was not the case when this experiment was conducted with auranofin (Figure 3B).

The mode of inhibition was further characterized by investigating the effect of substrate competition. In an enzymatic assay, which depends on the interaction between TRXR1 and its natural substrate TRX1, the inventors observed that increasing amounts of TRX1 reduced enzyme inhibition by DKFZ-608, an effect not observed with auranofin (Figure 3C).

In an alternative enzymatic assay, using DTNB as a small molecule substrate of TRXR1, the inventors also observed a right-shift of the dose response curve of DKFZ-608 upon substrate increase, indicating a competitive mechanism of TRXR1 inhibition (not shown). Auranofin, the irreversible control compound, showed no affect with increasing DTNB concentrations. The reversible binding mode was confirmed by plotting the data according to Lineweaver-Burke.

TRXR1 inhibition by DKFZ-608 was further demonstrated in living cells by using the TRXR-selective off-on fluorescent probe TRFS-green (Zhang et al., 2014). Interestingly, the inventors observed a more potent inhibition of TRXR1 in the SCLC line H209 as compared to the NSCLC line A549, with dose-response curves that were almost identical to those generated in cytotoxicity experiments with DKFZ-608 in the same cell lines (Figure 4A). Uptake experiments suggest that this is not due to differential drug import or retention. Rather, SCLC cells express significantly lower levels of TRX1 protein as compared to NSCLC lines (Figure 4B), a finding supported by microarray and RNA-SEQ data from CCLE. Therefore, the observed inhibition of TRXR1 in cells is in agreement with the biochemical data, in that enzyme inhibition inversely correlates with expression levels of TRX1. This was confirmed by induced overexpression of TRX1 in the SCLC line H69, where the inventors observed a rightshift of the dose response curves of cytotoxicity and TRXR1 inhibition, indicating a desensitisation upon TRX overexpression (Figure 4C).

DNA methylation analysis provides data which suggest that the TRX1 promoter might be suppressed by hypermethylation in SCLC cell lines, when compared to NSCLC lines. The inventors identified the largest differences in DNA methylation at positions -995 and -1418 in the distal promoter region, an area which has previously been shown to contain a response element for oxidative stress (Osborne et al., 2006) (Figure 4D).

These findings show that expression levels of TRX1 are a predictive biomarker for the in vivo response of lung tumour cells to dithiocarbamate compounds such as DKFZ-608. This notion is supported by in situ histochemical analysis of TRX expression in tumour samples in that one finds lower TRX1 levels in tumour sections from SCLC patients, as compared to NSCLC samples (Figure 4E).

### B: Improved Dimeric Dithiocarbamates:

### Example B1: Derivatives with -OH groups are more effective on Spheroid cultures.

Human SCLC cells (H209) were cultured either in flat bottom or V-bottom wells. While H209 cells grow in flat bottom wells as single suspension cells or small aggregates (< 10 cells/aggregate), they form spheroids in V-bottom wells. 50 000 cells seeded per well, form one huge spheroid.

DKFZ-608, like other cytostatic drugs, is very sensitive to spheroid formation and is less active under such conditions. The IC50 in spheroid cultures of H209 cells 10.9 µM as compared to 0,011 µM in suspension cultures. The "Spheroid Effect" (IC50 in spheroid cultures/IC50 in suspension cultures) was 1842. In contrast, for all tested compounds with an OH-group this factor was between 38 and 92 fold lower (see Table 1).

Due to the much lower Spheroid Effect, i.e. hindering of drug tumour cell interaction in highly compact tissues, OH-substituted AuDTCs are more useful for the treatment of solid tumours and single hidden tumour cells in normal tissue. The latter is specifically important for the eradication of standard therapy surviving cells that have the potential to initiate tumour recurrence.

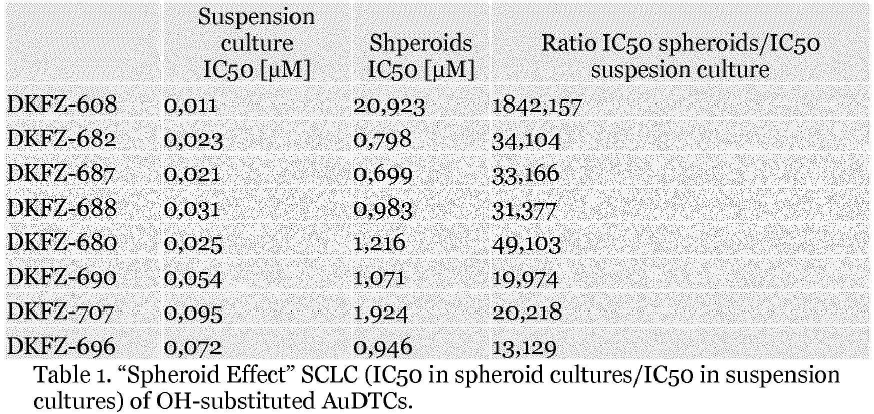

### Example B2: Derivatives with -OH groups are less affected by cell density.

Human SCLC cells (H209) were cultured in flat bottom well 96-well plates at various cell densities. Cells were treated with various concentrations of AuDTCs. After 72 h, the number of surviving cells was determined by CellTiterBlue staining. IC50 values for each AuDTC was determined from dose response curves.

Results: The non-substituted derivatives DKFZ-608 and DKFZ-693 showed a very strong reduction in activity at high cell densities. More than 100 fold higher IC50 values were found at 300 000 cells per well as compared 10 000 cells per well.

In contrast, the OH-substituted derivatives DKFZ-681 and DKFZ-682 were much less sensitive to cell density. The IC50 of DKFZ-682 was increased by a factor of 1.5 at 300 000 cells/well as compared to 10 000 cells/well.

Due to the much lower effect of cell density, OH-substituted AuDTCs are more useful for the treatment of solid tumours and single hidden tumour cells in normal tissue. The latter is specifically important for the eradication of standard therapy surviving cells that have the potential to initiate tumour recurrence. Results are provided in table 2:

**Table 2: IC50 values of candidate compounds on different cell densities in H209 cells**

| **Cells/well** | **10 000** | **30 000** | **100 000** | **300 000** | **Cell-density/effect** |
|---|---|---|---|---|---|
| **IC50 [µM]** | | | | | |
| DKFZ-682 | 0,012 | 0,012 | 0,023 | 0,018 | 1,562 |
| DKFZ-681 | 0,068 | 0,113 | 0,212 | 0,278 | 4,057 |
| DKFZ-693 | 0,067 | 0,173 | 0,238 | 93,031 | 1388,339 |
| DKFZ-608 | 0,019 | 0,034 | 0,063 | 79,554 | 4136,333 |

### Example B3: Derivatives with -OH groups induce cell death faster in lung cancer.

A549 lung cancer cells were treated with various OH-substituted Au^{I}-dtc complexes and the unsubstituted complex DKFZ-608 for various times. After incubation, cells were washed 2 x with PBS and fresh, inhibitor free medium was added. After 72 h in total (incubation time with test compound + inhibitor free incubation time) the number of surviving cells was determined by CellTiterBlue staining. IC50 values were determined from dose response curves.

DKFZ-608 shows an anti-tumoural effect only after an incubation time of at least 320 min. The OH-substituted derivatives DKFZ-687, DKFZ-707 and DKFZ-682, showed anti-tumoural effects already after 10, 20 and 40 min respectively. Most notably, the DKFZ-682 induced cell death already after 80 min with very high efficiency, while its enantiomer DKFZ-687 showed highest efficiency only after 4320 min.

As most anti-tumour drugs have short plasma half-lifes in the range of 1h or even lower, it is of great importance, that a drug excerts its antitumoural effect within such a short time. Surprisingly, this is the case with OH-substituted AuDTCs (Figure 5A).

### Example B4: Derivatives with -OH groups induce cell death faster in colon cancer.

HCT116 colon lung cancer cells were treated with various OH-substituted Au^{I}-dtc complexes and the unsubstituted complex DKFZ-608 for various times. After incubation, cells were washed 2 x with PBS and fresh, inhibitor free medium was added. After 72 h in total (incubation time with test compound + inhibitor free incubation time) the number of surviving cells was determined by CellTiterBlue staining. IC50 values were determined from dose response curves.

DKFZ-608 shows an anti-tumoural effect only after an incubation time of at least 2440 min. The OH-substituted derivatives effective after, DKFZ-688, DKFZ-690, and DKFZ-707 showed anti-tumoural effects already after 150 min. Most notably DKFZ-682 induced cell death already after 10 min (Figure 5B).

### Example B5: General Procedure for the Synthesis of Au^{I}-dtc complexes

The appropriate amine (0.92 mmol, 1.2 equiv. or 1.15 mmol, 1.5 equiv.) and potassium hydroxide (51.8 mg, 0.92 mmol, 1.2 equiv. or 64.5 mg, 1.15 mmol, 1.5 equiv.) were dissolved in 10.0 ml of *aqua dest.* (in case of employing amine hydrochlorides the double amount of potassium hydroxide was used) and carbon disulfide (70.3 mg, 0.92 mmol, 1.2 equiv. or 87.5 mg, 0.06 ml, 1.5 equiv.) was added dropwise. The solution was stirred at room temperature for 2h in order to assure complete *in situ* formation of the corresponding dithiocarbamate (dtc). Then sodium aurothiomalate (300mg, 0.77 mmol, 1.0 equiv.) predissolved in 15.0 ml of *aqua dest.* was added to the solution. A yellow to orange colored precipitate formed immediately. The suspension was stirred at room temperature over night, filtered through a glass frit (pore 4) and thoroughly washed with distilled water (2 x 20 ml). This material was dried under high vacuum over-night (and eventually recrystallized from an appropriate solvent) to afford the pure product.

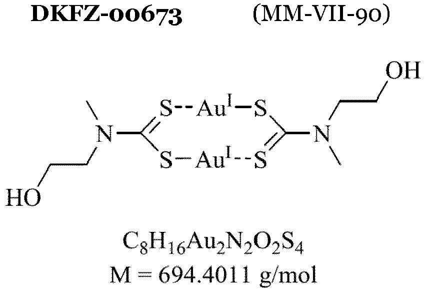

**[Au^{I}(*N*-2-hydroxyethyl, *N*-methyl)dtc]₂:** According to the general procedure using 2-methylaminoethanol (86.6 mg, 0.09 ml, 1.15 mmol, 1.5 equiv.) a greenish solid was obtained which was recrystallized from hot ethanol to afford small orange-red needles as the pure product in a yield of 73% (196.5 mg, 0.28 mmol).
**¹H NMR** (400 MHz, DMSO-*d*₆) δ 5.04 (t, *J* = 5.5 Hz, 2H), 4.02 (t, *J* = 5.7 Hz, 4H), 3.74 (q, *J* = 5.6 Hz, 4H), 3.49 (s, 6H).
**¹³C NMR** (100 MHz, DMSO-*d₆*) δ 201.39, 61.39, 57.88, 47.04.
**HR-ESI** (positive mode): m/z [Au^{III} + 2 dtc⁻]⁺ calcd. for C₈H₁₆AuN₂O₂S₄: 496.9760, found: 496.9757.
**Elemental analysis** (report 42262, [M]): calcd C, 13.84; H, 2.32; N, 4.03; obsd C, 13.76; H, 2.43; N, 3.98.

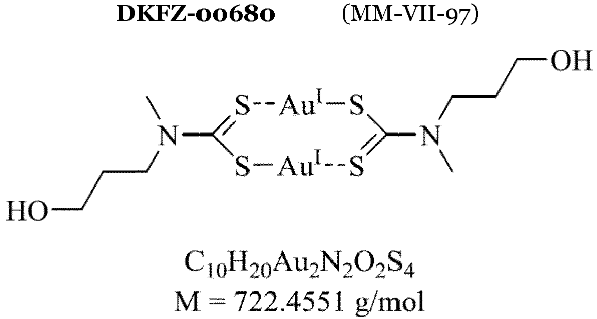

**[Au^{I}(*N*-3-hydroxyethyl, *N*-methyl)dtc]₂:** According to the general procedure using 3-methylamino-i-propanol (82.0 mg, 0.08 ml, 0.92 mmol, 1.2 equiv.) a greenish to brown solid was obtained as the pure product in a yield of 76% (210.9 mg, 0.29 mmol).
**¹H NMR** (400 MHz, DMSO-*d*₆) δ 4.63 (t, *J* = 5.0 Hz, 2H), 4.01 - 3.92 (m, 4H), 3.46 (d, *J* = 5.6 Hz, 10H), 1.93 - 1.81 (m, 4H).
**¹³C NMR** (100 MHz, DMSO-*d*₆) δ 200.85, 58.07, 57.03, 45.69, 29.48.
**HR-ESI** (positive mode): m/z [Au^{III} + 2 dtc⁻]⁺ calcd. for C₁₀H₂₀AuN₂O₂S₄: 525.0073, found: 525.0070.
**Elemental analysis** (report 42328, [M]): calcd C, 16.62; H, 2.79; N, 3.88; obsd C, 16.38; H, 2.88; N, 3.87.

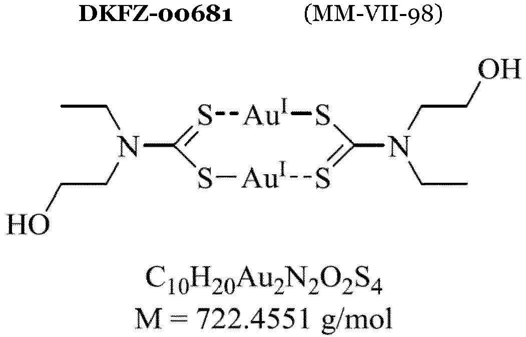

**[Au^{I}(*N*-ethyl, *N*-2-hydroxyethyl)dtc]₂:** According to the general procedure using 2-ethylaminoethanol (82.0 mg, 0.09 ml, 0.92 mmol, 1.2 equiv.) a bright red solid was obtained as the pure product in a yield of 44% (123.5 mg, 0.17 mmol).
**¹H NMR** (400 MHz, DMSO-*d*₆) δ 5.06 (t, *J* = 5.4 Hz, 2H), 3.99 (q, *J* = 7.2 Hz, 4H), 3.95 (t, *J* = 6.0 Hz, 4H), 3.77 (q, *J* = 5.7 Hz, 4H), 1.27 (t, *J* = 7.0 Hz, 6H).
**¹³C NMR** (100 MHz, DMSO-*d*₆) δ 200.95, 58.72, 57.81, 53.77, 11.23.
**HR-ESI** (positive mode): m/z [Au^{III} + 2 dtc⁻]⁺ calcd. for C₁₀H₂₀AuN₂O₂S₄: 525.0073, found: 525.0080.
**Elemental analysis** (report 42329, [M + H₂O]): calcd C, 16.22; H, 2.99; N, 3.78; obsd C, 16.15; H, 2.83; N, 3.64.

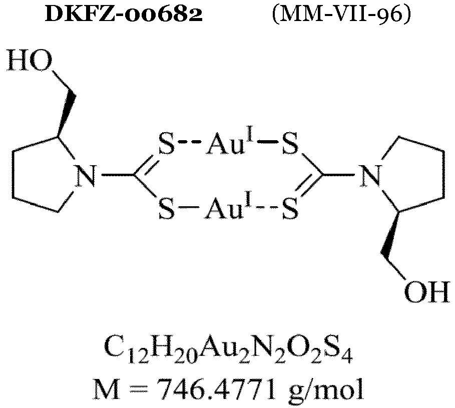

**[Au^{I}((*S*)-prolinoyl)dtc]₂:** According to the general procedure using (*S*)-(+)-prolinol (93.1 mg, 0.92 mmol, 1.2 equiv.) a pale-yellow solid was obtained which was recrystallized from hot ethanol (with a few drops of DMF) to afford red-brownish crystals as the pure product in a yield of 82% (236.8 mg, 0.32 mmol).
**¹H NMR** (400 MHz, DMSO-*d*₆) δ 5.11 - 5.01 (m, 2H), 4.54 - 4.44 (m, 2H), 3.81 - 3.74 (m, 4H), 3.74 - 3.52 (m, 4H), 2.24 - 1.93 (m, 8H).
**¹³C NMR** (100 MHz, DMSO-*d*₆) δ 198.21, 68.24, 59.02, 56.57, 27.66, 23.09.
**HR-ESI** (positive mode): m/z [Au^{III} + 2 dtc⁻]⁺ calcd. for C₁₂H₂₀AuN₂O₂S₄: 549.0073, found: 549.0070.
**Elemental analysis** (report 42327, [M + H₂O]): calcd C, 18.55; H, 2.90; N, 3.66; obsd C, 18.53; H, 2.93; N, 3.67.

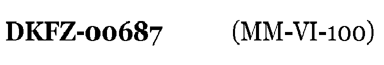

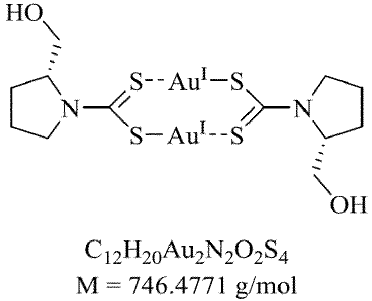

**[Au^{I}((*R*)-prolinoyl)dtc]₂:** According to the general procedure using (R)-(-)-prolinol (93.1 mg, 0.92 mmol, 1.2 equiv.) a yellow-golden solid was obtained as the pure product in a yield of 53% (150.6 mg, 0.20 mmol).
**¹H NMR** (400 MHz, DMSO-*d*₆) δ 5.06 (t, *J* = 5.7 Hz, 2H), 4.53 - 4.46 (m, 2H), 3.81 - 3.73 (m, 4H), 3.74 - 3.52 (m, 4H), 2.22 - 1.94 (m, 8H).
**¹³C NMR** (100 MHz, DMSO-*d*₆) δ 198.18, 68.25, 59.03, 56.58, 27.67, 23.10.
**Elemental analysis** (report 42366, [M]): calcd C, 19.31; H, 2.70; N, 3.75; obsd C, 19.30; H, 3.00; N, 3.75.

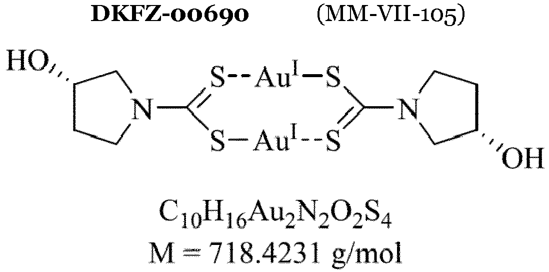

**[Au^{I}((*S*)-3-hydroxypyrrolidinyl)dtc]₂:** According to the general procedure using (*S*)-(+)-3-pyrrolidinol (80.2 mg, 0.92 mmol, 1.2 equiv.) a bright yellow solid was obtained as the pure product in a yield of 61% (167.6 mg, 0.23 mmol).
**Elemental analysis** (report 42369, [M + H₂O]): calcd C, 16.31; H, 2.46; N, 3.80; obsd C, 16.21; H, 2.46; N, 3.71.

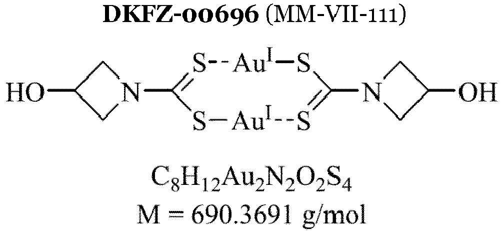

**[Au^{I}(3-hydroxyazetidinyl)dtc]₂:** According to the general procedure using 3-hydroxyazetidin hydrochloride (100.8 mg, 0.92 mmol, 1.2 equiv.) a yellow solid was obtained as the pure product in a yield of 66% (175.8 mg, 0.26 mmol).
**Elemental analysis** (report 42475, [M]): calcd C, 13.92; H, 1.75; N, 4.06; obsd C, 13.68; H, 1.97; N, 4.04.

## Claims

1. A method for diagnosing a tumour disease in a patient, the method comprising the steps of
(g) Detecting the level of thioredoxin 1 (TRX1) in a sample from the patient;
(h) Comparing the detected level of TRX1 in the sample from the patient with a reference value or with a level of TRX1 in a reference sample,
wherein a reduced level of TRX1 in the sample from the patient compared to the reference value or with the level of TRX1 in a reference sample indicates a tumour which is treatable with a competitive thioredoxin reductase 1 (TRXR1) inhibitor.

2. A method for deciding how to treat a tumour disease in a patient, the method comprising the steps of
(i) Detecting the level of thioredoxin 1 (TRX1) in a sample from the patient;
(j) Comparing the detected level of TRX1 in the sample from the patient with a reference value or with a level of TRX1 in a reference sample,
wherein a reduced level of TRX1 in the sample from the patient compared to the reference value or with the level of TRX1 in a reference sample indicates a treatment of the patient with a competitive thioredoxin reductase 1 (TRXR1) inhibitor.

3. The method according to claim 1 or 2, wherein the sample is a biological sample and obtained from the patient, preferably wherein the biological sample comprises at least one tumour cell of the tumour disease to be diagnosed.

4. The method according to any one of claims 1 to 3, wherein detecting the level of TRX1 in the sample involves detection of the level of protein and/or mRNA of TRX1, and/or the TRX1 promoter region methylation status.

5. The method according to claim 4, wherein the methylation in the promoter region is detected at CpG sites at positions -995, and /or -1418 distal to the translational start site (ATG) in the TRX1 gene, or to a region not more than 100bp, preferably 50bp, 40bp, or 20bp, preferably 10bp, most preferably 5bp away from positions -995, and /or -1418.

6. A competitive TRXR1 binder for use in a method of treating a patient suffering from a tumour disease, wherein the method comprises a step of administering to the patient an amount of the competitive TRXR1 binder, wherein the administered amount of the a competitive TRXR1 binder is calculated based on the detected level of TRX1 in a tumour cell of the tumour of the patient.

7. The competitive TRXR1 binder for use according to claim 6, wherein in first steps a sample of the tumour of the patient is analyzed for the level of TRX1, preferably the first steps comprising:
(k) Detecting the level of thioredoxin 1 (TRX1) in a sample from the patient;
(l) Comparing the detected level of TRX1 in the sample from the patient with a reference value or with a level of TRX1 in a reference sample.

8. The competitive TRXR1 binder for use according to claim 6 or 7, wherein the tumour is selected from a chemotherapy resistant tumour disease, and/or is a small cell lung cancer (SCLC) or lymphoma. a lung cancer, preferably is small cell lung cancer (SCLC).

9. A compound selected from a dimeric dithiocarbamate metal complex according to the following formula (I), or any solvates, salts, stereoisomers, polymorphs, crystalline forms, racemic mixtures, diastereomers, enantiomers, tautomers, isotopically labelled forms, prodrugs, and combinations thereof: 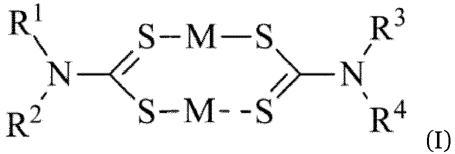
wherein R¹, R², R³ and ⁴ are the same or different, or where R¹ and R² and/or R³ and R⁴ a connected to form a 3- to 8-membered ring, and are selected from hydrogen, and unsubstituted or substituted C₁ to C₂₀ straight, branched or cyclic alkyl, unsubstituted or substituted C₁ to C₂₀ straight, branched or cyclic alkenyl, unsubstituted or substituted C₁ to C₂₀ alkynyl, unsubstituted or substituted aryl, and unsubstituted or substituted heteroaryl; and
wherein M is a metal ion;
**characterized in that**
R¹, R², R³ and/or R⁴ comprise at least one free hydroxyl group (-OH group).

10. The compound according to claim 9, wherein R¹ and R² do not form a ring; and wherein R¹ comprises at least one free hydroxyl group, and R² does not comprise a free hydroxyl group, or wherein R² comprises at least one free hydroxyl group, and R₁ does not comprise a free hydroxyl group.

11. The compound according to claim 9 or 10, wherein the at least one free hydroxyl group is provided in context of the any of the following groups:

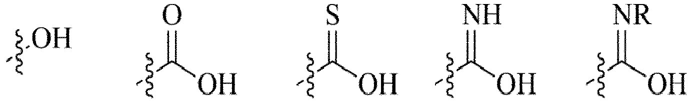

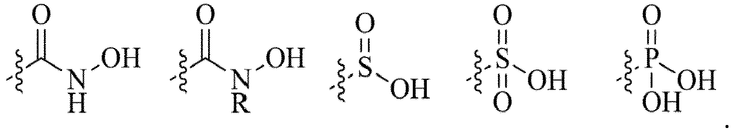

12. The compound according to any one of the preceding claims, wherein a substituent is selected from halogen, -CF₃, -C₂F₅, -C₃F₇, -C₄F₉, -C₅F₁₁ and other fluoroalkyl of 2 to 5 carbon atoms, -OH, -NH₂, -NO₂, -CHO, -CN, -COOH, -CONHOH, -SH, SO₂OH,-CONH₂, -NHNH₂, -OR, -NRR', -C(O)R, -C(O)OR, -C(O)NRR', -NR'C(O)R, -OC(O)R, aryl ring with 5 to 10 carbon atoms, cycloalk(en)yl rings with 3 to 20 carbon atoms, 3-to 10-membered heterocycloalk(en)yl rings, and 5- to 10-membered heteroaryl rings, wherein R and R' are independently selected from hydrogen, straight or branched alkyl chains with 1 to 10 carbon atoms, straight or branched alkenyl chains with 2 to 10 carbon atoms, straight or branched alkynyl chains with 2 to 10 carbon atoms, aryl rings with 5 to 14 carbon atoms, cycloalk(en)yl rings with 3 to 20 carbon atoms, 5- to 14-membered heteroaryl rings, comprising 1 to 4 heteroatoms selected from nitrogen, oxygen, sulfur and phosphor, and 5- to 14-membered heteroalk(en)yl rings, comprising 1 to 4 heteroatoms selected from nitrogen, oxygen, sulfur and phosphor, and optionally wherein said substituent is further substituted.

13. The compound according to any one of the preceding claims having any one of the following structures:

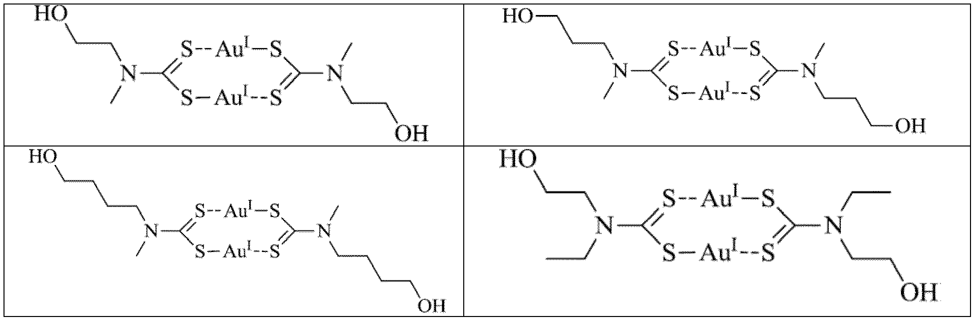

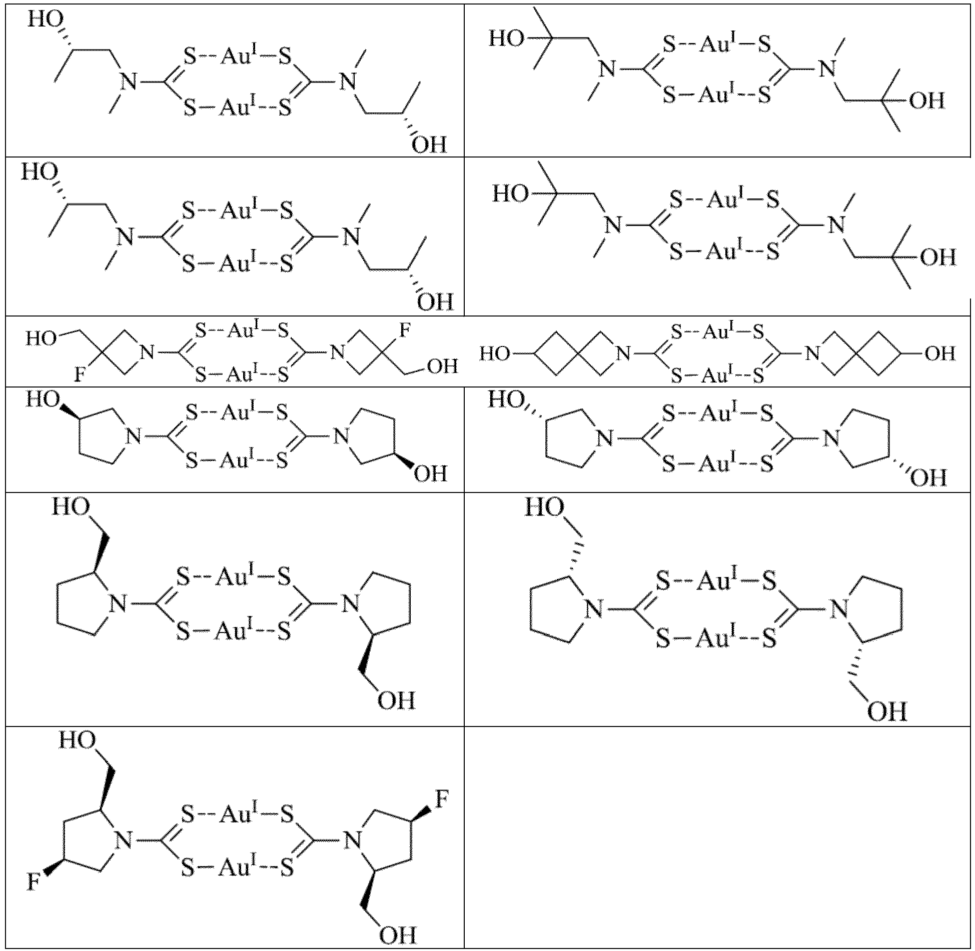

14. A method according to any one of claims 1 to 6, or a competitive TRXR1 binder according to claim 7 or 8 wherein the competitive TRXR1 binder is a compound according to any one of claims 9 to 13.

15. A pharmaceutical composition comprising the compound of any one of claims 9 to 13, and pharmaceutically acceptable carrier and/or excipient, optionally wherein the carrier and/or excipient includes cyclodextrin.

16. A product for use in the treatment of a disease, wherein the product is selected from a compound of any one of claims 9 to 13, or 14, or the pharmaceutical composition of claim 15, preferably wherein the disease is a proliferative disease, preferably a cancer disease, such as SCLC.
